# EUROPEAN PATENT SPECIFICATION

(11) **EP 2 900 244 B1**
(45) Date of publication and mention of the grant of the patent: **23.10.2019**
(21) Application number: 13840550.1
(22) Date of filing: 27.09.2013
(51) Int. Cl.: A61K 31/546, A61K 31/431, A61K 9/19, A61P 31/04, A61K 45/06

(54) **TAZOBACTAM ARGININE ANTIBIOTIC COMPOSITIONS**
ANTIBIOTISCHE ZUSAMMENSETZUNGEN AUS TAZOBACTAM-ARGININ
COMPOSITIONS ANTIBIOTIQUES À BASE D'ARGININE-TAZOBACTAME

(30) Priority: 27.09.2012 US 201261706399 P
(43) Date of publication of application: 05.08.2015
(73) Proprietor: Merck Sharp & Dohme Corp., Rahway, NJ 07065 (US)
(72) Inventor: LAI, Jan-Ji, Westborough, MA 01581 (US); GU, Jian-Qiao, Lexington, MA 02420 (US); PATHARE, Pradip, M., Lexington, MA 02421 (US); JURKAUSKAS, Valdas, Cambridge, MA 02138 (US); TERRACCIANO, Joseph, Concord, MA 01742 (US); MILLER DAMOUR, Nicole, Belmont, MA 02478 (US)
(74) Representative: Jaap, David Robert
(86) International application number: PCT/US2013/062256
(87) International publication number: WO 2014/052799

(56) References cited:
- EP-A1- 1 273 586
- WO-A1-95/12601
- RU-C2- 2 397 768
- US-A1- 2004 162 277
- DATABASE WPI Week 200015 Thomson Scientific, London, GB; AN 2000-161717 XP002755659, & CN 1 236 781 A (CHINA MEDICINE & BIOLOGIC PROD INSPECTIN) 1 December 1999 (1999-12-01)
- MORRIS K R ET AL: "An integrated approach to the selection of optimal salt form for a new drug candidate", INTERNATIONAL JOURNAL OF PHARMACEUTICS, ELSEVIER BV, NL, vol. 105, no. 3, 9 May 1994 (1994-05-09), pages 209-217, XP025565568, ISSN: 0378-5173, DOI: 10.1016/0378-5173(94)90104-X [retrieved on 1994-05-09]
- SADER HELIO S. ET AL.: 'Antimicrobial Activity of CXA-101, a Novel Caphalosporin Tested in Combination with Tazobactam against Enterobacteriaceae, Pseudomonas aeruginosa, and Bacteroides fragilis Strains Having Various Resistance Phenotypes.' ANTIMICROBIAL AGENTS AND CHEMOTHERAPY vol. 55, no. 5, May 2011, pages 2390 - 2394, XP055065385
- GHRISTIAN GISKE G. ET AL.: 'Activity of cephalosporin CXA-101 (FR264205) and comparators against extended-spectrum-B-lactamase-producing Pseudomonas aeruginosa' JOURNAL OF ANTIMICROBIAL CHEMOTHERAPY vol. 64, no. 2, 2009, pages 430 - 431, XP055065382

## Description

### TECHNICAL FIELD

This disclosure relates to pharmaceutical compositions as defined in the claims comprising tazobactam arginine and related methods and uses thereof.

### BACKGROUND

US 2004162277 concerns anhydrous crystals of tazobactam. EP 1273586 concerns a tazobactam diphenylmethyl ester crystal. CN1236781 concerns tazobactam semi-hydrate. WO9512601 concerns crystalline sodium tazobactam. Morris, K. R. et al., "An integrated approach to the selection of optimal salt form for a new drug candidate", International Journal of Pharmaceutics, vol. 105, no. 3, pages 209 - 217 concerns research into the optimization of BMS-180437.

The cephalosporin (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate (also referred to as ceftolozane, or (6R,7R)-3-[5-Amino-4-[3-(2-aminoethyl)ureido]-1-methyl-1H-pyrazol-2-ium-2-ylmethyl]-7-[2-(5-amino-1,2,4-thiadiazol-3-yl)-2-[(Z)-1-carboxy-1-methylethoxyimino]acetamido]-3-cephem-4-carboxylic acid) is an antibacterial agent. The antibacterial activity of ceftolozane is believed to result from its interaction with penicillin binding proteins (PBPs) to inhibit the biosynthesis of the bacterial cell wall which acts to stop bacterial replication. Ceftolozane can be combined (e.g., mixed) with a β-lactamase inhibitor ("BLI"), such as tazobactam. Tazobactam is a BLI against Class A and some Class C β-lactamases, with well-established *in vitro* and *in vivo* efficacy in combination with active β-lactam antibiotics.

Antibiotic pharmaceutical compositions can include a beta-lactam compound having antibiotic properties (i.e., an antibiotic compound possessing one or more beta-lactam moieties) and a BLI, such as tazobactam. Beta-lactam compounds can be formulated with and/or administered in combination with, beta-lactamase inhibiting compounds (e.g., tazobactam and salts thereof) in order to mitigate the effects of bacterial beta-lactamases. For example, the combination of ceftolozane and tazobactam in a 2:1 weight ratio is an antibiotic pharmaceutical composition ("CXA-201") formulated for parenteral administration. CXA-201 displays potent antibacterial activity *in vitro* against common Gram-negative and selected Gram-positive organisms. CXA-201 is a broad-spectrum antibacterial with *in vitro* activity against Enterobacteriaceae including strains expressing extended spectrum β-lactamases-resistant (MIC₉₀ = 1 µg/mL), as well as *Pseudomonas aeruginosa* (*P. aeruginosa*) including multi-drug resistant strains (MIC₉₀= 2 µg/mL). CXA-201 is a combination antibacterial with activity against many Gram-negative pathogens known to cause intrapulmonary infections, including nosocomial pneumonia caused by *P. aeruginosa.*

### SUMMARY

Provided herein are compositions comprising a beta-lactam compound which is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate or a pharmaceutically acceptable salt thereof (i.e. ceftolozane, or a pharmaceutically acceptable salt thereof) and crystalline tazobactam arginine, and pharmaceutical compositions prepared using the beta-lactam compound and crystalline tazobactam arginine. Methods of making and related uses of these combinations are also provided.

Particularly, pharmaceutical compositions comprise the beta-lactam compound and crystalline tazobactam arginine. Crystalline compounds of tazobactam arginine can also possess properties that are beneficial to the preparation of various drug formulations and pharmaceutical compositions. Pharmaceutical compositions comprising crystalline forms of tazobactam arginine, or pharmaceutical compositions prepared using crystalline forms of tazobactam arginine, may exhibit beneficial properties including desired levels of chemical stability over time and/or in the presence of heat and humidity, and reduced levels of impurities. Compared with previous crystalline forms of tazobactam, certain crystalline tazobactam arginine solid forms are provided herein that have the advantageous characteristic of being less hygroscopic. These crystalline tazobactam arginine solid forms can have good thermal stability and light stability in the process of preparation, packing, transportation and storage.

The beta-lactam compound used in combination with crystalline tazobactam arginine is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt thereof.

In another aspect, provided herein is a method of making a pharmaceutical composition comprising combining crystalline tazobactam arginine and the beta-lactam compound. In one embodiment, the method comprises the steps of: (1) preparing a mixture comprising crystalline tazobactam arginine and the beta-lactam compound; (2) preparing an aqueous solution from the mixture; and (3) lyophilizing the solution to obtain said pharmaceutical composition.

Also provided are pharmaceutical compositions prepared according to the above method.

The above pharmaceutical compositions can be for use in methods for the treatment of bacterial infections in a mammal, the methods comprising administering to said mammal a therapeutically effective amount of the pharmaceutical compositions.

### BRIEF DESCRIPTION OF THE DRAWINGS

**Figure 1** depicts the X-ray powder diffraction pattern of polymorph Ia.
**Figure 2** depicts the differential scanning calorimetry (DSC) thermogram of polymorph Ia.
**Figure 3** depicts the thermogravimetric analysis (TGA) curve of polymorph Ia.
**Figure 4** depicts the X-ray powder diffraction pattern of polymorph Ib.
**Figure 5** depicts impurities observed in Example 3.

### DETAILED DESCRIPTION

Pharmaceutical compositions comprising one or more drug substances or excipients can be prepared in a variety of ways, including, for example, blending and lyophilization (also known as "co-lyophilization"). As is known to those skilled in the art, lyophilization is a process of freeze-drying in which water is sublimed from a frozen solution of one or more solutes. Specific methods of lyophilization are described in Remington's Pharmaceutical Sciences, Chapter 84, page 1565, Eighteenth Edition, A. R. Gennaro, (Mack Publishing Co., Easton, Pa., 1990).

The formulation of pharmaceutical compositions can be selected to minimize decomposition of the constituent drug substances and to produce a composition that is stable under a variety of storage conditions. Surprisingly, pharmaceutical compositions comprising crystalline forms of tazobactam arginine (e.g., pharmaceutical compositions prepared using crystalline forms of tazobactam arginine) have been observed to exhibit beneficial properties including desired levels of chemical stability over the course of time and/or in the presence of heat and humidity, and reduced levels of impurities. In a particular embodiment (see Example 4), a pharmaceutical composition prepared from crystalline tazobactam arginine and ceftolozane was observed to undergo less decomposition of both tazobactam and ceftolozane over time.

The beneficial properties of the above pharmaceutical compositions may be attributable to the unique physical properties of crystalline tazobactam arginine. Tazobactam arginine can occur in an amorphous solid form or in a crystalline solid form. Crystalline solid forms of tazobactam arginine can exist in one or more unique polymorph forms, which can additionally comprise one or more equivalents of water or solvent (i.e., hydrates or solvates, respectively).

Tazobactam arginine is the salt of the conjugate base of tazobactam and the conjugate acid of (*S*)-2-amino-5-guanidinopentanoic acid (L-arginine) in a 1:1 ratio, as represented by the structure below.

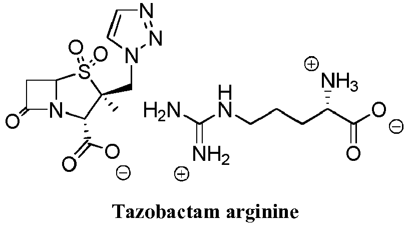

Accordingly, provided herein are compositions comprising a beta-lactam compound which is ceftolozane or a pharmaceutically acceptable salt thereof and crystalline tazobactam arginine, or hydrates and solvates thereof, particularly crystalline tazobactam arginine polymorph la, (also referred to herein as "polymorph Ia" or "tazobactam arginine polymorph Ia") and crystalline tazobactam arginine polymorph Ib (also referred to herein as "polymorph Ib" or "tazobactam arginine polymorph Ib").

### Polymorphism

The ability of a substance to exist in more than one crystal form is defined as polymorphism; the different crystal forms of a particular substance are referred to as "polymorphs." In general, polymorphism is affected by the ability of a molecule of a substance to change its conformation or to form different intermolecular or intra-molecular interactions, particularly hydrogen bonds, which is reflected in different atom arrangements in the crystal lattices of different polymorphs. In contrast, the overall external form of a substance is known as "morphology," which refers to the external shape of the crystal and the planes present, without reference to the internal structure. Crystals can display different morphology based on different conditions, such as, for example, growth rate, stirring, and the presence of impurities.

The different polymorphs of a substance can possess different energies of the crystal lattice and, thus, in solid state they can show different physical properties such as form, density, melting point, color, stability, solubility, dissolution rate, etc., which can, in turn, affect the stability, dissolution rate and/or bioavailability of a given polymorph and its suitability for use as a pharmaceutical and in pharmaceutical compositions.

Access to different polymorphs of tazobactam arginine is desirable for other reasons as well. One such reason is that different polymorphs of a compound (e.g., tazobactam arginine) can incorporate different impurities, or chemical residues, upon crystallization. Certain polymorphs incorporate very little, or no, chemical residues. Accordingly, the formation of certain polymorph forms of a compound may result in purification of the compound.

Tazobactam arginine polymorph Ia exhibits low hygroscopicity relative to amorphous tazobactam arginine and amorphous tazobactam sodium. Low hygroscopicity of a solid compound is desirable for several reasons. For example, compounds that are highly hygroscopic may be chemically unstable, or unsuitable for formulating as a drug product due to changes of the drug form's physical characteristics (e.g., bulk density, dissolution rate, etc.) that can occur if it is stored in settings with varying relative humidity. Also, hygroscopicity can impact large-scale manufacturing and handling of a compound. For example, it may be difficult to determine the true weight of a hygroscopic active agent when preparing a pharmaceutical composition comprising that agent.

### Characterization of solid crystalline forms of tazobactam arginine

In certain embodiments, the compounds used in the combination therapies described herein are identifiable on the basis of characteristic peaks in an X-ray powder diffraction analysis. X-ray powder diffraction, also referred to as XRPD, is a scientific technique using X-ray, neutron, or electron diffraction on powder, microcrystalline, or other solid materials for structural characterization of the materials.

As used herein, the phrase "degrees 2-Theta ± 0.3°" indicates that each subsequently listed angle has an error of ± 0.3°; the phrase "degrees 2-Theta ± 0.2°" indicates that each subsequently listed angle has an error of ± 0.2°; and the phrase "degrees 2-Theta ± 0.1°" indicates that each subsequently listed angle has an error of ± 0.1°. For example, the phrase "degrees 2-Theta ± 0.2° at angles of 1, 2 and 3" is equivalent to the phrase "degrees 2-Theta at angles of 1 ± 0.2°, 2 ± 0.2° and 3 ± 0.2°".

One embodiment of crystalline tazobactam arginine used in the combination therapies described herein is referred to as polymorph Ia (also referred to herein as "tazobactam arginine polymorph Ia") and is characterized by an X-ray powder diffraction pattern having one or more characteristic peaks expressed in degrees 2-Theta at angles selected from about 8.9° ± 0.3°, about 18.0° ± 0.3° and about 21.2° ± 0.3°. In another embodiment, polymorph Ia is characterized by an X-ray powder diffraction pattern having one or more peaks expressed in degrees 2-Theta at angles selected from about 4.8° ± 0.3°, about 11.3° ± 0.3° and about 14.9° ± 0.3°. In still another embodiment, polymorph Ia is characterized by an X-ray powder diffraction pattern having one or more peaks expressed in degrees 2-Theta at angles selected from about 19.4° ± 0.3°, about 22.8° ± 0.3° and about 24.3° ± 0.3°.

In another embodiment, polymorph Ia is characterized by an X-ray powder diffraction pattern having 3-6 peaks expressed in degrees 2-Theta at angles selected from about 8.9° ± 0.3°, about 18.0° ± 0.3°, about 21.2° ± 0.3°, about 4.8° ± 0.3°, about 11.3° ± 0.3°, about 14.9° ± 0.3°, about 19.4° ± 0.3°, about 22.8° ± 0.3° and about 24.3° ± 0.3°. In a particular embodiment, polymorph Ia is characterized by an X-ray powder diffraction pattern having characteristic peaks expressed in degrees 2-Theta at angles of about 8.9° ± 0.3°, about 18.0° ± 0.3° and about 21.2° ± 0.3°.

In another embodiment, polymorph Ia is characterized by an X-ray powder diffraction pattern having 3-6 peaks expressed in degrees 2-Theta at angles selected from about 8.9° ± 0.3°, about 18.0° ± 0.2°, about 21.2° ± 0.2°, about 4.8° ± 0.2°, about 11.3° ± 0.2°, about 14.9° ± 0.2°, about 19.4° ± 0.2°, about 22.8° ± 0.2° and about 24.3° ± 0.2°. In a particular embodiment, polymorph Ia is characterized by an X-ray powder diffraction pattern having characteristic peaks expressed in degrees 2-Theta at angles of about 8.9° ± 0.2°, about 18.0° ± 0.2° and about 21.2° ± 0.2°.

In yet another embodiment, polymorph Ia is characterized by an X-ray powder diffraction pattern having 6-9 peaks expressed in degrees 2-Theta at angles selected from about 8.9° ± 0.3°, about 18.0° ± 0.3°, about 21.2° ± 0.3°, about 4.8° ± 0.3°, about 11.3° ± 0.3°, about 14.9° ± 0.3°, about 19.4° ± 0.3°, about 22.8° ± 0.3° and about 24.3° ± 0.3°. In a particular embodiment, polymorph Ia is characterized by an X-ray powder diffraction pattern having characteristic peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°.

In yet another embodiment, polymorph Ia is characterized by an X-ray powder diffraction pattern having 6-9 peaks expressed in degrees 2-Theta at angles selected from about 8.9° ± 0.2°, about 18.0° ± 0.2°, about 21.2° ± 0.2°, about 4.8° ± 0.2°, about 11.3° ± 0.2°, about 14.9° ± 0.2°, about 19.4° ± 0.2°, about 22.8° ± 0.2° and about 24.3° ± 0.2°. In a particular embodiment, polymorph Ia is characterized by an X-ray powder diffraction pattern having characteristic peaks expressed in degrees 2-Theta at angles of about 4.8° ± 0.2°, about 8.9° ± 0.2°, about 11.3° ± 0.2°, about 14.9° ± 0.2°, about 18.0° ± 0.2°, about 19.4° ± 0.2°, about 21.2° ± 0.2° about 22.8° ± 0.2° and about 24.3° ± 0.2°.

In still another embodiment, provided herein is a composition comprising crystalline tazobactam arginine characterized by an X-ray powder diffraction pattern having peaks expressed in degrees 2-Theta ± 0.3° at angles of 4.8°, 8.9°, 11.3°, 14.9°, 18.0°, 19.4°, 21.2°, and 22.8°.

In still another embodiment, provided herein is a composition comprising crystalline tazobactam arginine characterized by an X-ray powder diffraction pattern having peaks expressed in degrees 2-Theta ± 0.2° at angles of 4.8°, 8.9°, 11.3°, 14.9°, 18.0°, 19.4°, 21.2°, and 22.8°.

In still another embodiment, provided herein is a composition comprising crystalline tazobactam arginine characterized by an X-ray powder diffraction pattern having peaks expressed in degrees 2-Theta ± 0.1° at angles of 4.8°, 8.9°, 11.3°, 14.9°, 18.0°, 19.4°, 21.2°, and 22.8°.

In still another embodiment, provided herein is a composition comprising crystalline tazobactam arginine characterized by an X-ray powder diffraction pattern having peaks expressed in degrees 2-Theta at angles of about 4.8°, 8.9°, 11.3°, 14.9°, 18.0°, 19.4°, 21.2°, and 22.8°.

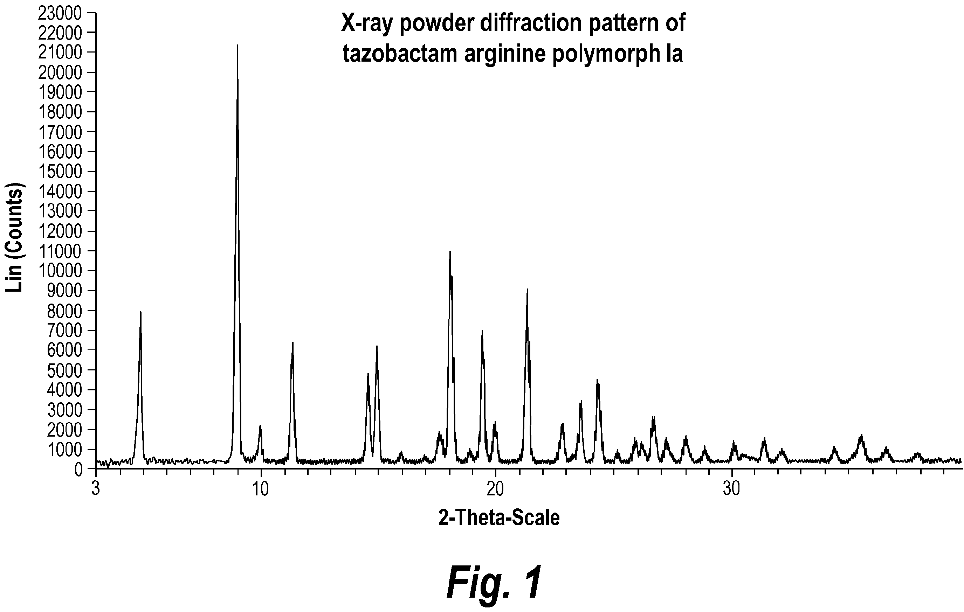

In one embodiment, polymorph Ia is characterized by an X-ray powder diffraction pattern having peaks substantially in accordance with Figure 1. In another embodiment, polymorph Ia is characterized by an X-ray powder diffraction pattern having peaks substantially in accordance with Table 1.

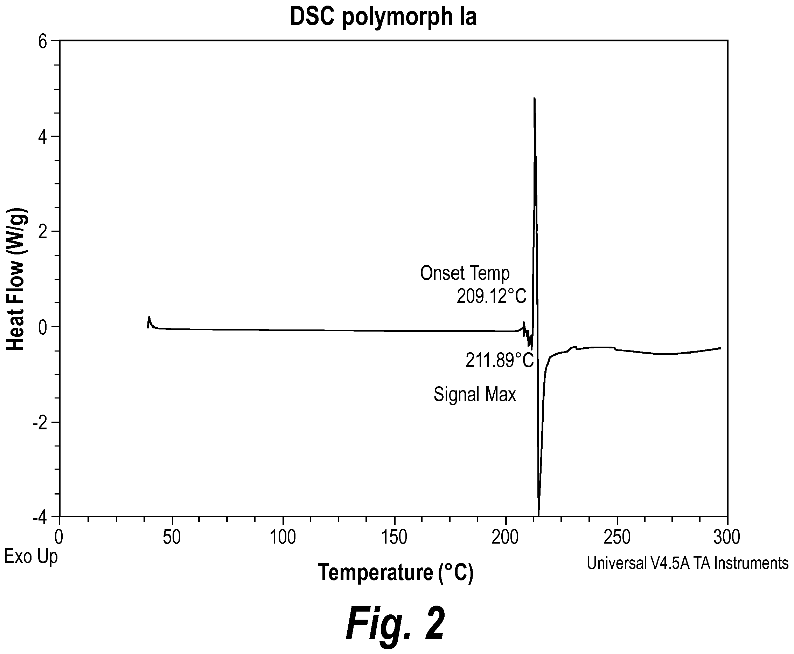

The compounds used in the combination therapies described herein may also be defined by their differential scanning calorimetry (DSC) thermograms. In one embodiment, polymorph Ia is characterized by a differential scanning calorimetry thermogram having a characteristic peak expressed in units of °C at a temperature of 209.2 ± 3. In another embodiment, polymorph Ia is characterized by a differential scanning calorimetry thermogram having a characteristic peak expressed in units of °C in the range of about 209.2 to about 211.9. In a particular embodiment, polymorph Ia is characterized by a differential scanning calorimetry thermogram substantially in accordance with Figure 2.

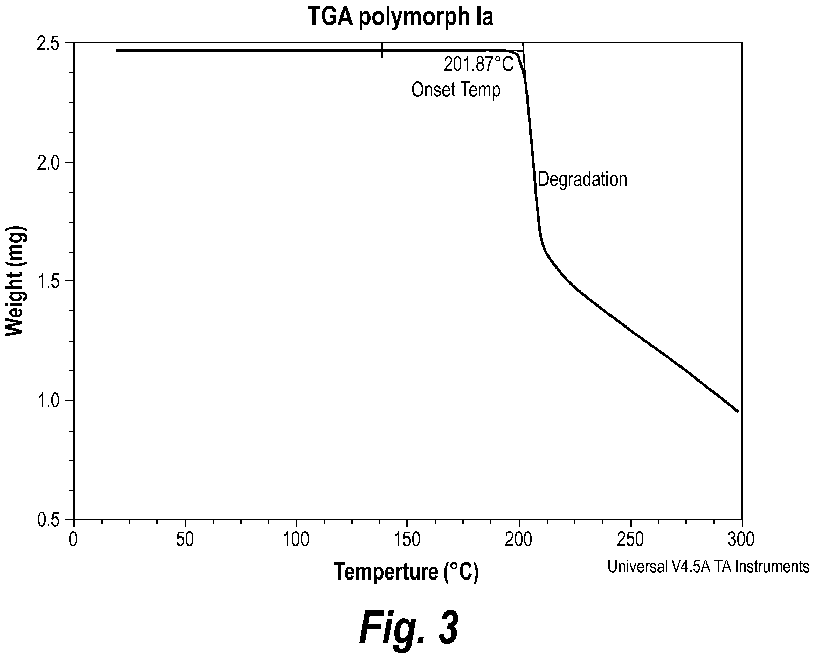

The compounds used in the combination therapies described herein can be also be defined by their thermogravimetry (TG) signals. In one embodiment, polymorph Ia is characterized by a thermogravimetry curve with an onset temperature of 201.8 °C ± 3 °C. In another embodiment, polymorph Ia is characterized by a thermogravimetry curve with an onset temperature of about 201.8 °C. In a particular embodiment, polymorph Ia is characterized by a thermogravimetry curve substantially in accordance with Figure 3.

In certain embodiments, polymorph Ia may contain impurities. Non-limiting examples of impurities include undesired polymorph forms, or residual organic and inorganic molecules such as solvents, water or salts.

In another embodiment, polymorph Ia is substantially free from impurities. In another embodiment, polymorph Ia contains less than 10% by weight total impurities. In another embodiment, polymorph Ia contains less than 5% by weight total impurities. In another embodiment, polymorph Ia contains less than 1% by weight total impurities. In yet another embodiment, polymorph Ia contains less than 0.1% by weight total impurities.

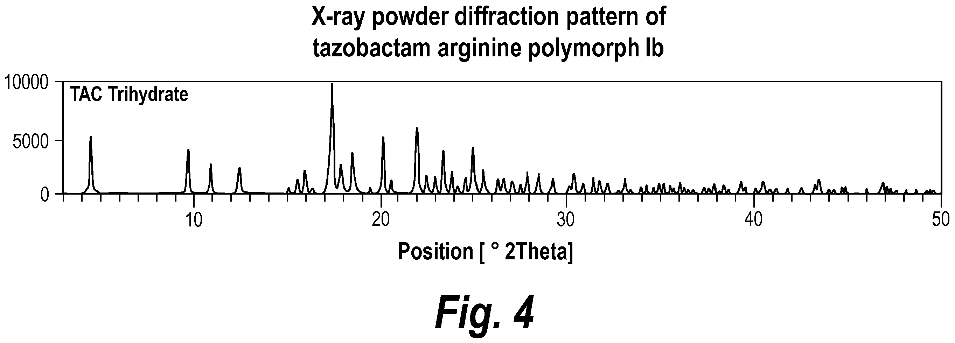

In another aspect, provided herein is crystalline tazobactam arginine polymorph Ib. In one embodiment, polymorph Ib is tazobactam arginine trihydrate. In another embodiment, crystalline tazobactam polymorph lb is characterized by an X-ray powder diffraction pattern having peaks expressed in degrees 2-Theta at angles of about 4.4° ± 0.3°, about 9.7° ± 0.3°, about 17.3° ± 0.3°, about 20.2° ± 0.3°, and about 22.0° ± 0.3°. In a particular embodiment, polymorph lb is characterized by an X-ray powder diffraction pattern having peaks substantially in accordance with Figure 4.

In another aspect, provided herein is a combination comprising a beta-lactam compound which is ceftolozane or a pharmaceutically acceptable salt thereof and a composition comprising one or more compounds selected from crystalline tazobactam arginine, polymorph Ia and polymorph lb. In one embodiment, the composition comprises one or more compounds selected from crystalline tazobactam arginine and polymorph Ia.

In certain embodiments, polymorph Ia is a crystalline solid substantially free of amorphous tazobactam arginine. As used herein, the term "substantially free of amorphous tazobactam arginine" means that the compound contains no significant amount of amorphous tazobactam arginine. In certain embodiments, at least about 95% by weight of crystalline polymorph Ia is present. In still other embodiments of the invention, at least about 99% by weight of crystalline polymorph Ia is present.

In another embodiment, polymorph Ia is substantially free from polymorph lb. As used herein, the term "substantially free of polymorph Ib" means that the compound contains no significant amount of polymorph Ib. In certain embodiments, at least about 95% by weight of crystalline polymorph Ia is present. In still other embodiments of the invention, at least about 99% by weight of crystalline polymorph Ia is present.

### Beta-lactam compounds

A "beta-lactam compound" is a compound possessing one or more beta-lactam moieties, i.e., 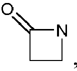 substituted one or more times as valency permits. Described herein are beta-lactam compounds that are antibacterial compounds. The beta-lactam compounds described herein can be selected from the group consisting of penicillins, cephalosporins, carbapenems, and combinations thereof. The beta-lactam compounds described herein are selected from the compounds listed in Table 2, and pharmaceutically acceptable isomers, salts, esters, hydrates, solvates, or combinations thereof. The following compounds are listed in Table 2:
- (2S,5R,6R)-6-[(R)-2-(4-ethyl-2,3-dioxo-1-piperazinecarboxamido)-2-phenylacetamido]-3,3-dimethyl-7-oxo-4-thia-1-azabicyclo[3.2.0]heptane-2-carboxylic acid;
- (2S,5R,6R)-3,3-dimethyl-7-oxo-6-(2-phenylacetamido)-4-thia-1-zabicyclo[3.2.0]heptane-2-carboxylic acid;
- (5R,6S)-6-[(1R)-1-hydroxyethyl]-3-({2-[(iminomethyl)amino]ethyl}thio)-7-oxo-1-azabicyclo[3.2.0]hept-2-ene-2-carboxylic acid;
- (5R,6S)-6-((R)-1-hydroxyethyl)-7-oxo-3-((R)-tetrahydrofuran-2-yl)-4-thia-1-azabicyclo[3.2.0]hept-2-ene-2-carboxylic acid;
- (2S,5R,6R)-6-{[3-(2-chlorophenyl)-5-methyl-oxazole-4-carbonyl]amino}-3,3-dimethyl-7-oxo-4-thia-1-azabicyclo[3.2.0]heptane-2-carboxylic acid;
- (6R,7R,Z)-7-(2-(2-aminothiazol-4-yl)-2-(2-carboxypropan-2-yloxyimino)acetamido)-8-oxo-3-(pyridinium-1-ylmethyl)-5-thia-1-aza-bicyclo[4.2.0] oct-2-ene-2-carboxylate; (6R,7R,Z)-3-(acetoxymethyl)-7-(2-(2-aminothiazol-4-yl)-2-(methoxyimino)acetamido)-8-oxo-5-thia-1-azabicyclo[4.2.0]oct-2-ene-2-carboxylic acid;
- (6R,7R)-7-[(2Z)-2-ethoxyimino-2-[5-(phosphonoamino)-1,2,4-thiadiazol-3-yl]acetyl]amino]-3-[4-(1-methylpyridin-1-ium-4-yl)-1,3-thiazol-2-yl]sulfanyl]-8-oxo-5-thia-1-azabicyclo[4.2.0]oct-2-ene-2-carboxylate;
- (6R,7R,Z)-7-(2-(2-aminothiazol-4-yl)-2-(methoxyimino)acetamido)-3-((1-methylpyrrolidinium-1-yl)methyl)-8-oxo-5-thia-1-aza-bicyclo[4.2.0]oct-2-ene-2-carboxylate;
- (6R,7R)-3-{[(aminocarbonyl)oxy]methyl}-7-{[(2Z)-2-(2-furyl)-2-(methoxyimino) acetyl]amino}-8-oxo-5-thia-1-azabicyclo[4.2.0]oct-2-ene-2-carboxylic acid;
- (6R,7R)-7-{[(2Z)-2-(2-amino-1,3-thiazol-4-yl)-2-(methoxyimino)acetyl]amino}-3-{[(2-methyl-5,6-dioxo-1,2,5,6-tetrahydro-1,2,4-triazin-3-yl)thio]methyl}-8-oxo-5-thia-1-azabicyclo[4.2.0]oct-2-ene-2-carboxylic acid;
- (2S,5R,6R)- 6-{[(2R)-2-amino-2-(4-hydroxyphenyl)-acetyl]amino}-3,3-dimethyl-7-oxo-4-thia-1-azabicyclo[3.2.0]heptane-2-carboxylic acid;
- 3-[5-(dimethylcarbamoyl) pyrrolidin-2-yl] sulfanyl-6-(1-hydroxyethyl)-4-methyl-7-oxo-1-azabicyclo[3.2.0] hept-2-ene-2-carboxylic acid;
- (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate; and
- 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl} -1 -methyl-1*H*-pyrazolium monosulfate.

The skilled practitioner will recognize that the beta-lactam compounds described herein have one or more acidic moieties (e.g., carboxylic acid moieties) and/or one or more basic moieties (e.g., amine moieties). Said moieties may be protonated or deprotonated as a function of pKa or pKb of the moiety and the pH of the compound's environment. All salt forms resulting from the protonation or deprotonation of a beta-lactam compound are contemplated by the instant disclosure.

Any beta-lactam compound, exemplified by those listed above, can be used in the pharmaceutical compositions described herein.

The compound 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate (also known also as ceftolozane sulfate) is a cephalosporin compound (shown below), the synthesis of which is described in U.S. Patent No. 7,129,232. As provided herein, ceftolozane can be in its free base form, or in the form of a pharmaceutically acceptable salt thereof, e.g., ceftolozane sulfate:

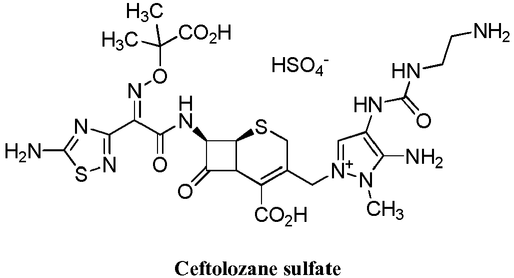

### Pharmaceutical Compositions

The term "pharmaceutical composition" includes preparations suitable for administration to mammals, e.g., humans. When the compounds of the present invention are administered as pharmaceuticals to mammals, e.g., humans, they can be given per se or as a pharmaceutical composition containing, for example, 0.1% to 99.9% (more preferably, 0.5 to 90%) of active ingredient in combination with a pharmaceutically acceptable carrier.

The pharmaceutical compositions described herein can be formulated to have any concentration desired (i.e., any concentration of crystalline tazobactam arginine, or a hydrate or solvate thereof, and any concentration of the beta-lactam compound). In some embodiments, the composition is formulated such that it comprises at least a therapeutically effective amount of both compounds (i.e., a therapeutically effective amount of the combination of crystalline tazobactam arginine, or a hydrate or solvate thereof, and the beta-lactam compound). In some embodiments, the composition is formulated such that it would not cause one or more unwanted side effects.

Pharmaceutical compositions include those suitable for oral, sublingual, nasal rectal, vaginal, topical, buccal and parenteral (including subcutaneous, intramuscular, and intravenous) administration, although the most suitable route will depend on the nature and severity of the condition being treated. The compositions may be conveniently presented in unit dosage form, and prepared by any of the methods well known in the art of pharmacy. In certain embodiments, the pharmaceutical composition is formulated for oral administration in the form of a pill, capsule, lozenge or tablet. In other embodiments, the pharmaceutical composition is in the form of a suspension.

Pharmaceutical compositions may additionally comprise excipients, stabilizers, pH adjusting additives (e.g., buffers) and the like. Non-limiting examples of these additives include sodium chloride, citric acid and L-arginine. For example, in the formulations of Example 2 and Example 3, the use of sodium chloride results in greater stability; L-arginine is used to adjust pH and to increase the solubility of ceftolozane; and citric acid is used prevent discoloration of the product, due to its ability to chelate metal ions.

The pharmaceutical compositions disclosed herein can be prepared via lyophilization (including, for example, co-lyophilization of more than one drug substances).

In a particular embodiment, the pharmaceutical compositions described herein are formulated for parenteral administration. In another particular embodiment, the pharmaceutical compositions described herein are formulated for administration by intravenous injection or infusion.

In one aspect, provided herein is a pharmaceutical composition comprising crystalline tazobactam arginine and a beta-lactam compound which is (6R,7R)-3-[(5-amino-4-{[(2-aminoethyl)carbamoyl] amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt thereof.

In another embodiment, the crystalline tazobactam arginine used in the combination therapies described herein is characterized by an X-ray powder diffraction pattern having one or more characteristic peaks expressed in degrees 2-Theta at angles of about 8.9° ± 0.3°, about 18.0° ± 0.3° and about 21.2° ± 0.3°. In yet another embodiment, the crystalline tazobactam arginine is characterized by an X-ray powder diffraction pattern having peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°.

In another embodiment, the crystalline tazobactam arginine used in the combination therapies described herein is characterized by an X-ray powder diffraction pattern having one or more characteristic peaks expressed in degrees 2-Theta at angles of about 8.9° ± 0.2°, about 18.0° ± 0.2° and about 21.2° ± 0.2°. In yet another embodiment, the crystalline tazobactam arginine is characterized by an X-ray powder diffraction pattern having peaks expressed in degrees 2-Theta at angles of about 4.8° ± 0.2°, about 8.9° ± 0.2°, about 11.3° ± 0.2°, about 14.9° ± 0.2°, about 18.0° ± 0.2°, about 19.4° ± 0.2°, about 21.2° ± 0.2° about 22.8° ± 0.2° and about 24.3° ± 0.2°.

In still another embodiment, the crystalline tazobactam arginine is characterized by a differential scanning calorimetry thermogram having a characteristic peak expressed in units of °C at a temperature in the range of about 209.2 to about 211.9. In still another embodiment, the crystalline tazobactam arginine is characterized by a thermogravimetry curve with an onset temperature of about 201.9 °C.

In a particular embodiment, the pharmaceutical composition comprises polymorph Ia and (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt thereof, and a pharmaceutically acceptable carrier or diluent. In a preferred embodiment, the pharmaceutical composition comprises polymorph Ia and 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

In another aspect, provided herein are pharmaceutical compositions prepared according to the following methods.

### Methods of making pharmaceutical compositions

Provided herein is a method of making a pharmaceutical composition, comprising combining crystalline tazobactam arginine and a beta-lactam compound which is ceftolozane or a pharmaceutically acceptable salt thereof. In one embodiment, the method comprises the steps of: (1) preparing a mixture comprising crystalline tazobactam arginine and the beta-lactam compound; (2) preparing an aqueous solution from the mixture; and (3) lyophilizing the solution to obtain said pharmaceutical composition. In one embodiment, the method further comprises reconstituting the lyophilized mixture in an aqueous solvent, such that the resulting solution is suitable for parenteral administration.

The crystalline tazobactam arginine is characterized as described above. For example, in one embodiment of the method, the crystalline tazobactam arginine is characterized by an X-ray powder diffraction pattern having one or more characteristic peaks expressed in degrees 2-Theta at angles selected from about 8.9° ± 0.3°, about 18.0° ± 0.3° and about 21.2° ± 0.3°. In another embodiment, the crystalline tazobactam arginine is characterized by an X-ray powder diffraction pattern having one or more characteristic peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°. In yet another embodiment, the crystalline tazobactam arginine is characterized by a differential scanning calorimetry thermogram having a characteristic peak expressed in units of °C at a temperature in the range of about 209.2 to about 211.9. In still another embodiment, the crystalline tazobactam arginine is characterized by a thermogravimetry curve with an onset temperature of about 201.9 °C.

The beta-lactam compound is (6R,7R)-3-[(5-amino-4-{[(2-aminoethyl)carbamoy]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt thereof. In a particular embodiment, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

In one embodiment of the method, and above embodiments, the molar ratio of crystalline tazobactam arginine to the beta-lactam compound in the mixture is in the range of 1:3 to 3:1. In another embodiment, the molar ratio of crystalline tazobactam arginine to the beta-lactam compound in the mixture is in the range of 1:2 to 2:1. In another embodiment, the molar ratio of crystalline tazobactam arginine to the beta-lactam compound in the mixture is in the range of 1:0.9 to 0.9:1. In a particular embodiment, the ratio of crystalline tazobactam arginine to the beta-lactam compound in the mixture is about 0.9:1. In another particular embodiment, the ratio of crystalline tazobactam arginine to the beta-lactam compound in the mixture is about 1:2.

In some embodiments, the mixture of crystalline tazobactam arginine and ceftolozane further comprises one or more additives selected from the group consisting of L-arginine, citric acid, and sodium chloride. In one embodiment, the molar ratio of L-arginine to the beta-lactam compound in the mixture is in the range of 4:1 to 1:4. In another embodiment, the molar ratio of L-arginine to the beta-lactam compound in the mixture is in the range of 3:1 to 1:3. In another embodiment, the molar ratio of L-arginine to the beta-lactam compound in the mixture is in the range of 2:1 to 1:2. In another embodiment, the molar ratio of L-arginine to the beta-lactam compound in the mixture is in the range of about 4:1 to about 2:1. In a particular embodiment, the molar ratio of L-arginine to the beta-lactam compound in the mixture is about 1.9:1.

In another embodiment of the method, the concentration of the beta-lactam compound in the aqueous solution is in the range of 0.01M - 10M. In another embodiment, the concentration of the beta-lactam compound in the aqueous solution is in the range of 0.01M - 1M. In a particular embodiment, the concentration of the beta-lactam compound in the aqueous solution is about 0.05M.

In still another embodiment of the method, the aqueous solution has a pH in the range of 5-7. In another embodiment, the aqueous solution has a pH in the range of 5.5-6.5. In a particular embodiment, the aqueous solution has a pH of about 6.3.

In another embodiment, ceftolozane (in free base or salt form, preferably hydrogen sulfate form) and tazobactam arginine are in a 2:1 (ceftolozane: tazobactam arginine) weight ratio, wherein the weight ratio is calculated based on the weight of ceftolozane in its free base, not salt, form. For example, a dose of the antibiotic composition comprising 300 mg ceftolozane hydrogen sulfate and 150 mg tazobactam arginine comprises an amount of ceftolozane hydrogen sulfate that corresponds to 300 mg of ceftolozane in its free base form.

In yet another embodiment, ceftolozane (in free base or salt form, preferably hydrogen sulfate form) and tazobactam arginine are in a 2:1 (ceftolozane: tazobactam) weight ratio, wherein the weight ratio is calculated based on the weights of ceftolozane and tazobactam in their free base, not salt, form. Accordingly, in a particular embodiment, the pharmaceutical composition comprises crystalline tazobactam arginine and ceftolozane sulfate in a ratio corresponding to one weight equivalent of tazobactam free base and two weight equivalents of ceftolozane free base.

### Methods of Treatment

Tazobactam arginine inhibits or decreases the activity of beta-lactamases (e.g., bacterial beta-lactamases), and can be combined with beta-lactam compounds (e.g., antibiotics), thereby broadening the spectrum of the beta-lactam compound and increasing the beta-lactam compound's efficacy against organisms that produce beta-lactamase. A compound or a composition possesses efficacy against an organism if it kills or weakens the organism, or inhibits or prevents reproduction the organism.

In one aspect, provided herein is a pharmaceutical composition prepared according to the methods described herein for use in a method for the treatment of bacterial infections in a mammal, comprising administering to said mammal a therapeutically effective amount of a pharmaceutical composition prepared according to the methods described herein. In another aspect, provided herein is crystalline tazobactam arginine and ceftolozane or a pharmaceutically acceptable salt thereof for use in a method for the treatment of bacterial infections in a mammal, comprising administering to said mammal a therapeutically effective amount of a crystalline tazobactam arginine and ceftolozane or a pharmaceutically acceptable salt thereof. In certain embodiments, the bacterial infection is caused by an extended-spectrum beta-lactamase-producing organism. In certain embodiments, the bacterial infection is caused by an antibiotic-resistant organism.

In another aspect, provided herein is a pharmaceutical composition comprising crystalline tazobactam arginine and ceftolozane or a pharmaceutically acceptable salt thereof for use in method for the treatment of bacterial infections in a mammal, comprising administering to said mammal a therapeutically effective amount of a pharmaceutical composition comprising crystalline tazobactam arginine and ceftolozane or a pharmaceutically acceptable salt thereof. In one embodiment, the mammal is human. In another embodiment, the crystalline tazobactam arginine is polymorph Ia.

In a particular embodiment of the method, the pharmaceutical composition comprises polymorph Ia and 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

In another aspect, provided herein is a pharmaceutical composition comprising crystalline tazobactam arginine and ceftolozane or a pharmaceutically acceptable salt thereof for use in a method for the treatment of bacterial infections in a mammal, comprising administering to said mammal a therapeutically effective amount of a pharmaceutical composition comprising ceftolozane or a pharmaceutically acceptable salt thereof and a crystalline tazobactam arginine compound (e.g., of the polymorph Ia solid form). The crystalline tazobactam arginine can be characterized by an X-ray powder diffraction pattern having peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°. The crystalline tazobactam arginine can also be characterized by an X-ray powder diffraction pattern having peaks expressed in degrees 2-Theta at angles of about 4.8° ± 0.2°, about 8.9° ± 0.2°, about 11.3° ± 0.2°, about 14.9° ± 0.2°, about 18.0° ± 0.2°, about 19.4° ± 0.2°, about 21.2° ± 0.2° about 22.8° ± 0.2° and about 24.3° ± 0.2°.

Non-limiting examples of bacterial infections that can be treated by the invention include infections caused by: aerobic and facultative gram-positive microorganisms (e.g., *Staphylococcus aureus, Enterococcus faecalis, Staphylococcus epidermidis, Streptococcus agalactiae, Streptococcus pneumonia, Streptococcus pyogenes, Viridans group streptococci),* aerobic and facultative gram-negative microorganisms (e.g., *Acinetobacter baumanii, Escherichia coli, Haemophilus influenza, Klebsiella pneumonia, Pseudomonas aeruginosa, Citrobacter koseri, Moraxella catarrhalis, Morganella morganii, Neisseria gonorrhoeae, Proteus mirabilis, Proteus vulgaris, Serratia marcescens, Providencia stuartii, Providencia rettgeri, Salmonella enterica*), gram-positive anaerobes (*Clostridium perfringens*), and gram-negative anaerobes (e.g., *Bacteroides fragilis group* (e.g., *B. fragilis, B. ovatus, B. thetaiotaomicron, and B. vulgates*), *Bacteroides distasonis, Prevotella melaninogenica*).

In certain embodiments, bacterial infection resulting from beta-lactamase-producing organisms are treated or controlled. Non-limiting examples of beta-lactamase-producing organisms include:
(1) ESBL (extended-spectrum beta-lactamase)-producing organisms selected from the group consisting of *Enterobacteriaceae spp.: Escherichia coli, Klebsiella spp.* (including *K. pneumoniae* and *K. oxytoca*), *Proteus mirabilis, Proteus vulgaris, Enterobacter spp., Serratia spp., Citrobacter spp., Pseudomonas spp., Acinetobacter spp*.) and *Bacteroides spp.;*
(2) CSBL (conventional-spectrum beta-lactamase)-producing organisms, known to those of skill in the art; and
(3) Inducible-AmpC-type beta-lactamases, such as *Citrobacter spp., Serratia spp., Morganella morganii, Proteus vulgaris,* and *Enterobacter cloacae.*

In certain embodiments, bacterial infection is associated with one or more of the following conditions:
Appendicitis (complicated by rupture or abscess) and peritonitis caused by piperacillin-resistant beta-lactamase producing strains of *Escherichia coli* or the following members of the Bacteroides fragilis group: *B. fragilis, B. ovatus, B. thetaiotaomicron,* or *B. vulgates;*
Uncomplicated and complicated skin and skin structure infections, including cellulitis, cutaneous abscesses, and ischemic/diabetic foot infections caused by piperacillin-resistant, beta-lactamase producing strains of *Staphylococcus aureus;*
Postpartum endometritis or pelvic inflammatory disease caused by piperacillin-resistant, beta-lactamase producing strains of *Escherichia coli;*
Community-acquired pneumonia (moderate severity only) caused by piperacillin-resistant, beta-lactamase producing strains of *Haemophilus influenza;*
Nosocomial pneumonia (moderate to severe) caused by piperacillin-resistant, beta-lactamase producing strains of *Staphylococcus aureus* and by *Acinetobacter baumanii, Haemophilus influenzae, Klebsiella pneumoniae, and Pseudomonas aeruginosa.*

Complicated intra-abdominal infections; Complicated urinary tract infections (cUTIs); Acute Pyelonephritis; Systemic Inflammatory Response Syndrome (SIRS).

Also described herein is the use of a crystalline tazobactam arginine, and hydrates and solvates thereof, in combination with one or more beta-lactam compounds, for the preparation of a medicament for the treatment of bacterial infection. The bacterial infection can result from either gram-negative or gram-positive organisms. The crystalline tazobactam arginine is polymorph Ia. Polymorph Ia is characterized as described above. Said one or more beta-lactam compounds can be selected from the group consisting of penicillins, cephalosporins, carbapenems, and combinations thereof. Said one or more beta-lactam compounds are selected from the compounds listed in Table 2, and pharmaceutically acceptable isomers, salts, esters, hydrates, solvates, or combinations thereof.

In one aspect, the invention provides crystalline tazobactam arginine and a beta-lactam compound which is ceftolozane or a pharmaceutically acceptable salt thereof for use in a method of treating a bacterial infection in a mammal. In one embodiment, the crystalline tazobactam arginine and beta-lactam compound are parenterally administered. Typically, the crystalline tazobactam arginine and beta-lactam compound are intravenously administered. In some embodiments, the crystalline tazobactam arginine and beta-lactam compound are administered as an infusion.

In one embodiment, the crystalline tazobactam arginine and beta-lactam compound which is ceftolozane or a pharmaceutically acceptable salt thereof are for use in a method of treating a bacterial infection in a mammal, wherein the bacterial infection is caused by an extended-spectrum beta-lactamase-producing organism. In another embodiment, the crystalline tazobactam arginine and beta-lactam compound are for use in a method of treating a bacterial infection in a mammal, wherein the bacterial infection is caused by an antibiotic-resistant organism. In a preferred embodiment, the crystalline tazobactam arginine and beta-lactam compound are for use in a method of treating a complicated urinary tract infection. In another preferred embodiment, the crystalline tazobactam arginine and beta-lactam compound are for use in a method of treating a complicated intra-abdominal infection. In a further preferred embodiment, the crystalline tazobactam arginine and beta-lactam compound are for use in a method of treating nosocomial pneumonia. The crystalline tazobactam arginine and beta-lactam compound may be for use in a method of treating ventilator acquired pneumonia or hospital acquired pneumonia.

The beta-lactam compound is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt thereof. In a particularly preferred embodiment, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

In one preferred embodiment, the crystalline tazobactam arginine is tazobactam arginine polymorph Ia. The crystalline tazobactam arginine may be characterized by an X-ray powder diffraction pattern having one or more characteristic peaks expressed in degrees 2-Theta at angles of about 8.9° ± 0.3°, about 18.0° ± 0.3° and about 21.2° ± 0.3°. The crystalline tazobactam arginine may be characterized by an X-ray powder diffraction pattern having peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°. In some embodiments, the crystalline tazobactam arginine is characterized by a differential scanning calorimetry thermogram having a characteristic peak expressed in units of °C at a temperature in the range of about 209.2 to about 211.9. The crystalline tazobactam arginine may be characterized by a thermogravimetry curve with an onset temperature of about 201.9 °C.

In the most preferred embodiments, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate and the crystalline tazobactam arginine is tazobactam arginine polymorph Ia.

In one aspect, the invention provides crystalline tazobactam arginine for use in a method of treating a bacterial infection in a mammal, comprising administration of crystalline tazobactam arginine in combination with a beta-lactam compound which is ceftolozane or a pharmaceutically acceptable salt thereof. In one embodiment, the crystalline tazobactam arginine and/or beta-lactam compound is parenterally administered. Typically, the crystalline tazobactam arginine and/or beta-lactam compound is intravenously administered. In some embodiments, the crystalline tazobactam arginine and/or beta-lactam compound is administered as an infusion. In one embodiment, both the crystalline tazobactam arginine and beta-lactam compound are parenterally administered. In one embodiment, both the crystalline tazobactam arginine and beta-lactam compound are intravenously administered. In another embodiment, both the crystalline tazobactam arginine and beta-lactam compound are administered as an infusion.

In one embodiment, the crystalline tazobactam arginine is for use in a method of treating a bacterial infection in a mammal, wherein the bacterial infection is caused by an extended-spectrum beta-lactamase-producing organism. In another embodiment, the crystalline tazobactam arginine is for use in a method of treating a bacterial infection in a mammal, wherein the bacterial infection is caused by an antibiotic-resistant organism. In a preferred embodiment, the crystalline tazobactam arginine is for use in a method of treating a complicated urinary tract infection. In another preferred embodiment, the crystalline tazobactam arginine is for use in a method of treating a complicated intra-abdominal infection. In a further preferred embodiment, the crystalline tazobactam arginine is for use in a method of treating nosocomial pneumonia. The crystalline tazobactam arginine may be for use in a method of treating ventilator acquired pneumonia or hospital acquired pneumonia.

The beta-lactam compound is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt thereof. In a particularly preferred embodiment, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

In one preferred embodiment, the crystalline tazobactam arginine is tazobactam arginine polymorph Ia. The crystalline tazobactam arginine may be characterized by an X-ray powder diffraction pattern having one or more characteristic peaks expressed in degrees 2-Theta at angles of about 8.9° ± 0.3°, about 18.0° ± 0.3° and about 21.2° ± 0.3°. The crystalline tazobactam arginine may be characterized by an X-ray powder diffraction pattern having peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°. In some embodiment, the crystalline tazobactam arginine is characterized by a differential scanning calorimetry thermogram having a characteristic peak expressed in units of °C at a temperature in the range of about 209.2 to about 211.9. The crystalline tazobactam arginine may be characterized by a thermogravimetry curve with an onset temperature of about 201.9 °C.

In the most preferred embodiments, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate and the crystalline tazobactam arginine is tazobactam arginine polymorph Ia.

In one aspect, the invention provides a beta-lactam compound which is ceftolozane or a pharmaceutically acceptable salt thereof for use in a method of treating a bacterial infection in a mammal, comprising administration of the beta-lactam compound in combination with crystalline tazobactam arginine. In one embodiment, the beta-lactam compound and/or crystalline tazobactam arginine is parenterally administered. Typically, the beta-lactam compound and/or crystalline tazobactam arginine is intravenously administered. In some embodiments, the beta-lactam compound and/or crystalline tazobactam arginine is administered as an infusion. In one embodiment, both the beta-lactam compound and crystalline tazobactam arginine are parenterally administered. In one embodiment, both the beta-lactam compound and crystalline tazobactam arginine are intravenously administered. In another embodiment, both the beta-lactam compound and crystalline tazobactam arginine are intravenously administered as an infusion.

In one embodiment, the beta-lactam compound is for use in a method of treating a bacterial infection in a mammal, wherein the bacterial infection is caused by an extended-spectrum beta-lactamase-producing organism. In another embodiment, the beta-lactam compound is for use in a method of treating a bacterial infection in a mammal, wherein the bacterial infection is caused by an antibiotic-resistant organism. In a preferred embodiment, the beta-lactam compound is for use in a method of treating a complicated urinary tract infection. In another preferred embodiment, the beta-lactam compound is for use in a method of treating a complicated intra-abdominal infection. In a further preferred embodiment, the beta-lactam compound is for use in a method of treating nosocomial pneumonia. The beta-lactam compound may be for use in a method of treating ventilator acquired pneumonia or hospital acquired pneumonia.

The beta-lactam compound is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt thereof. In a particularly preferred embodiment, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

In one preferred embodiment, the crystalline tazobactam arginine is tazobactam arginine polymorph Ia. The crystalline tazobactam arginine may be characterized by an X-ray powder diffraction pattern having one or more characteristic peaks expressed in degrees 2-Theta at angles of about 8.9° ± 0.3°, about 18.0° ± 0.3° and about 21.2° ± 0.3°. The crystalline tazobactam arginine may be characterized by an X-ray powder diffraction pattern having peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°. In some embodiment, the crystalline tazobactam arginine is characterized by a differential scanning calorimetry thermogram having a characteristic peak expressed in units of °C at a temperature in the range of about 209.2 to about 211.9. The crystalline tazobactam arginine may be characterized by a thermogravimetry curve with an onset temperature of about 201.9 °C.

In the most preferred embodiments, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate and the crystalline tazobactam arginine is tazobactam arginine polymorph Ia.

In one aspect, the invention provides crystalline tazobactam arginine and a beta-lactam compound which is ceftolozane or a pharmaceutically acceptable salt thereof as a combined preparation for simultaneous, separate or sequential use in a method of treating a bacterial infection in a mammal. In one embodiment, the crystalline tazobactam arginine and beta-lactam compound are parenterally administered. Typically, the crystalline tazobactam arginine and beta-lactam compound are intravenously administered. In some embodiments, the crystalline tazobactam arginine and beta-lactam compound are administered as an infusion.

In one embodiment, the crystalline tazobactam arginine and beta-lactam compound are for use in a method of treating a bacterial infection in a mammal, wherein the bacterial infection is caused by an extended-spectrum beta-lactamase-producing organism. In another embodiment, the crystalline tazobactam arginine and beta-lactam compound are for use in a method of treating a bacterial infection in a mammal, wherein the bacterial infection is caused by an antibiotic-resistant organism. In a preferred embodiment, the crystalline tazobactam arginine and beta-lactam compound are for use in a method of treating a complicated urinary tract infection. In another preferred embodiment, the crystalline tazobactam arginine and beta-lactam compound are for use in a method of treating a complicated intra-abdominal infection. In a further preferred embodiment, the crystalline tazobactam arginine and beta-lactam compound are for use in a method of treating nosocomial pneumonia. The crystalline tazobactam arginine and beta-lactam compound may be for use in a method of treating ventilator acquired pneumonia or hospital acquired pneumonia.

The beta-lactam compound is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable isomer, salt thereof. In a particularly preferred embodiment, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

In one preferred embodiment, the crystalline tazobactam arginine is tazobactam arginine polymorph Ia. The crystalline tazobactam arginine may be characterized by an X-ray powder diffraction pattern having one or more characteristic peaks expressed in degrees 2-Theta at angles of about 8.9° ± 0.3°, about 18.0° ± 0.3° and about 21.2° ± 0.3°. The crystalline tazobactam arginine may be characterized by an X-ray powder diffraction pattern having peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°. In some embodiment, the crystalline tazobactam arginine is characterized by a differential scanning calorimetry thermogram having a characteristic peak expressed in units of °C at a temperature in the range of about 209.2 to about 211.9. The crystalline tazobactam arginine may be characterized by a thermogravimetry curve with an onset temperature of about 201.9 °C.

In the most preferred embodiments, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate and the crystalline tazobactam arginine is tazobactam arginine polymorph Ia.

In one aspect, the invention provides crystalline tazobactam arginine and a beta-lactam compound which is ceftolozane or a pharmaceutically acceptable salt thereof for use in therapy. In one embodiment, the crystalline tazobactam arginine and beta-lactam compound are parenterally administered. Typically, the crystalline tazobactam arginine and beta-lactam compound are intravenously administered. In some embodiments, the crystalline tazobactam arginine and beta-lactam compound are administered as an infusion.

The beta-lactam compound is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt thereof. In a particularly preferred embodiment, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

In one preferred embodiment, the crystalline tazobactam arginine is tazobactam arginine polymorph Ia. The crystalline tazobactam arginine may be characterized by an X-ray powder diffraction pattern having one or more characteristic peaks expressed in degrees 2-Theta at angles of about 8.9° ± 0.3°, about 18.0° ± 0.3° and about 21.2° ± 0.3°. The crystalline tazobactam arginine may be characterized by an X-ray powder diffraction pattern having peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°. In some embodiments, the crystalline tazobactam arginine is characterized by a differential scanning calorimetry thermogram having a characteristic peak expressed in units of °C at a temperature in the range of about 209.2 to about 211.9. The crystalline tazobactam arginine may be characterized by a thermogravimetry curve with an onset temperature of about 201.9 °C.

In the most preferred embodiments, the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate and the crystalline tazobactam arginine is tazobactam arginine polymorph Ia.

As used herein, "treating", "treat" or "treatment" describes the management and care of a patient for the purpose of combating a disease, condition, or disorder and includes the administration of a pharmaceutical composition of the present invention to alleviate the symptoms or complications of a disease, condition or disorder, or to eliminate the disease, condition or disorder. The term "treat" can also include treatment of a cell in vitro or an animal model.

By a "therapeutically effective amount" of a compound of the invention is meant a sufficient amount of the compound to treat the disorder (e.g., bacterial infection). The specific therapeutically effective amount that is required for the treatment of any particular patient or organism (e.g., a mammal) will depend upon a variety of factors including the disorder being treated and the severity of the disorder; the activity of the specific compound or composition employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts (see, for example, Goodman and Gilman's, "The Pharmacological Basis of Therapeutics", Tenth Edition, A. Gilman, J.Hardman and L. Limbird, eds., McGraw-Hill Press, 155-173, 2001). The therapeutically effective amount for a given situation can be readily determined by routine experimentation and is within the skill and judgment of the ordinary clinician.

### Assays

Provided herein is a method for detecting or identifying an agent that will inhibit one or more beta-lactamase-producing organisms, said method comprising combining:
(a) a test agent;
(b) a composition comprising one or more beta-lactamase-producing organisms; and
(c) a beta-lactamase inhibitor; and detecting or measuring a change in the activity of the beta-lactamase-producing organisms, wherein a decrease in the activity of the beta-lactamase-producing organisms indicates that the test agent inhibits the beta-lactamase-producing organisms.

As used in the above method, "activity" refers to the ability of the beta-lactamase-producing organism to reproduce and/or infect another organism, or "activity" refers to the presence of an indicator of the ability of the beta-lactamase-producing organism to reproduce and/or infect another organism. Methods for detecting and/or measuring changes in the activity of beta-lactamase-producing organisms are known to those of skill in the art.

In another aspect, provided herein is a method of determining the susceptibility of a beta-lactamase-producing organism to a composition comprising a beta-lactam compound which is ceftolozane or a pharmaceutically acceptable salt thereof and a beta-lactamase inhibitor which is crystalline tazobactam arginine. The in vitro activity of compositions of the subject invention may be assessed by standard testing procedures. Non-limiting examples of such a procedure include the Kirby-Bauer method, the Stokes test, the E-test, broth dilution and agar dilution for determination of minimum inhibitory concentration (MIC), as described in "Approved Standard. Methods for Dilution Antimicrobial Susceptibility Tests for Bacteria that Grow Aerobically," 3.sup.rd ed., published 1993 by the National Committee for Clinical Laboratory standards, Villanova, Pa., USA. In certain embodiments, the methods described herein are performed using automation (e.g., Siemens' MicroScan Systems).
In a preferred embodiment, the beta-lactamase inhibitor is tazobactam arginine polymorph Ia.

### Instrumentation and Methods

I. X-Ray Powder Diffraction (XRPD) experiments were performed using a Bruker D8 Advance X-ray powder diffractometer utilizing a zero return silicon plate, a step size of 0.01°, a step time of 0.3 sec/step, Cu/Kα radiation, tube power of 40kV/40mA, a nickel filter, and a LynxEye high speed detector. A suitable amount of sample was placed directly on the sample holder, pressed flat to smooth, and analyzed from 3°-40° 2θ using Bragg-Brentano optics. Analysis was started immediately following sample preparation.
II. Differential Scanning Calorimetry (DSC) experiments were performed on a TA Instruments Q100 instrument. A temperature range of 40 °C to 300 °C with a ramp rate of 10 °C/minute was utilized. Approximately 1.0 mg of sample was weighed into a tared aluminum sample pan and sealed hermetically. A small hole was pushed into the cover of the sample pan to allow for pressure release.
III. Thermo Gravemetric Analysis (TGA) experiments were performed on a TA Instruments 5000 instrument from 20 to 300 °C with a heating rate of 10 °C/minute for all samples.

### Examples

Examples not falling within the scope of the claims are for reference only.

### Example 1: Preparation of Tazobactam Arginine Crystalline Polymorph Ia

Tazobactam arginine amorphous (1.00 g) was dissolved in 10.0 mL of deionized water. 30 mL of acetone was added to the aqueous solution by drop-wise addition. The mixture was allowed to sit overnight at ambient temperature, resulting in white fine needles. After filtration and vacuum drying for 4 hours, tazobactam arginine polymorph Ia (516 mg) was obtained. The XRPD spectrum of the tazobactam arginine polymorph Ia is depicted in Figure 1.

### Example 2: Preparation of pharmaceutical composition using tazobactam arginine polymorph Ia and ceftolozane.

A mixture is prepared comprising: tazobactam arginine polymorph Ia and ceftolozane in a molar ratio in the range of 1:2 to 2:1; L-arginine, such that the molar ratio of L-arginine to ceftolozane is in the range of 4:1 to 1:4; citric acid, such that the pH of an aqueous solution of the mixture is in the range of 5-7; and sodium chloride, such that the concentration of sodium chloride in an aqueous solution of the mixture is in the range of 0.1M - 1 M. The mixture is dissolved in deionized water, such that the molar ratio of ceftolozane in the aqueous solution is in the range of 0.01M - 10M. The resulting aqueous solution is then lyophilized to afford the title pharmaceutical composition.

### Example 3: Stability of Formulations of ceftolozane and solid forms of tazobactam

Formulations A-D of Table 3 were prepared as follows:
Formulation A: 1.237 g (1.5 mmol) of 90% ceftolozane sulfate, 0.62 g (3.56 mmol) of L-arginine, 0.022 g (0.115 mmol) of citric acid, 0.49 g (8.39 mmol) of NaCl was dissolved in 30 mL of water (final pH 5.81), then filtered through a 0.2 µm membrane, and lyophilized 24 hr to obtain an off-white powder, 2.2 g. A 480 mg portion was used for stability testing at 25 °C (60% RH).
Formulation B: 1.237 g (1.5 mmol) of 90% ceftolozane sulfate, 0.93 g (5.34 mmol) of L-arginine, 0.022 g (0.115 mmol) of citric acid, 0.50 g (1.67 mmol) of tazobactam acid, and 0.49 g (8.39 mmol) of NaCl was dissolved in 30 mL of water (final pH 6.72), then filtered through a 0.2 µm membrane, and lyophilized 24 hr to obtain an off-white powder, 3.22 g. A 490 mg portion was used for stability testing at 25 °C (60% RH).
Formulation C: 1.237 g (1.5 mmol) of 90% ceftolozane sulfate, 0.62 g (3.56 mmol) of L-arginine, 0.022 g (0.115 mmol) of citric acid, and 0.49 g (8.39 mmol) of NaCl was dissolved in 30 mL of water (resulting pH 6.34), then added 0.79 g (1.67 mmol) of tazobactam arginine polymorph Ia and stirred to dissolve (final pH 6.30), filtered through a 0.2 µm membrane, and lyophilized 24 hr to obtain an off-white powder, 3.10 g. A 510 mg portion was used for stability testing at 25 °C (60% RH).
Formulation D: 1.0 g of Formulation A (0.7 mmol ceftolozane sulfate; 1.67 mmol L-arginine), and 0.21 g (0.65 mmol) tazobactam sodium was dissolved in 20 mL of water (final pH 5.89), then filtered through a 0.2 µm membrane, and lyophilized 24 hr to obtain an off white-powder, 1.074 g. A 195 mg portion was tested for stability at 25 C (60% RH).

The above formulations were analyzed by HPLC at the following time points: T0: (Immediately after lyophilization); T1 (After one month at 25 °C and 60% relative humidity); and T2 (After three months at 25 °C and 60% relative humidity).

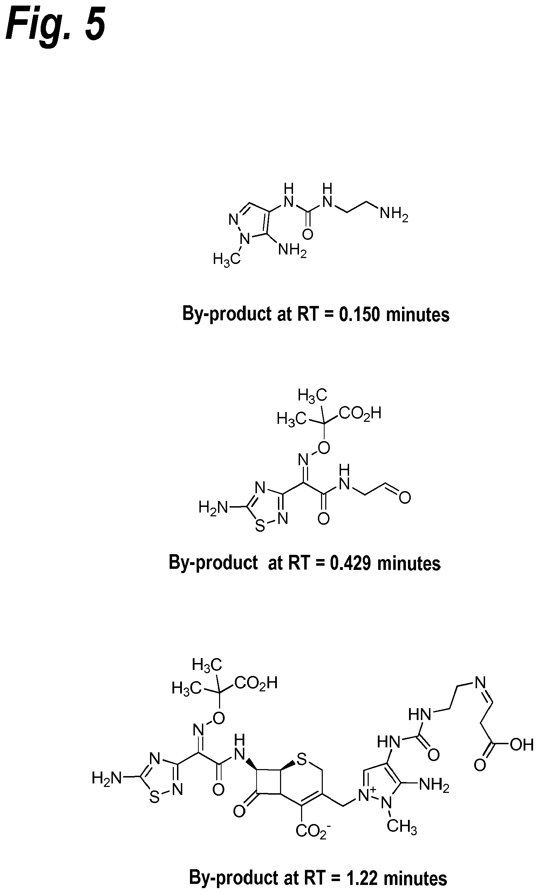

Of the three tazobactam-containing formulations (B, C and D), formulation D (containing tazobactam sodium) exhibited the highest degree of ceftolozane decomposition at T2. Formulation B (containing tazobactam acid and L-arginine) exhibited less ceftolozane decomposition than formulation D, and formulation C (containing tazobactam arginine polymorph Ia) exhibited significantly less ceftolozane decomposition than formulation B. Formulation C also exhibited significantly lower amounts of by-products having retention times of 0.150, 0.429 and 1.22 minutes, shown in Figure 5. These results are summarized in Table 4.

### Tables

**Table 1: XRPD Scanning Data of Tazobactam Arginine Polymorph Ia (Figure 1)**

| Chord Mid. 2-Theta ° | D (Obs. Max) Angstrom | Intensity % % | Max Int. Cps | Intensity Count | I. Breadth 2-Theta ° |
|---|---|---|---|---|---|
| 4.818 | 18.27951 | 33.5 | 130 | 7043 | 0.166 |
| 8.978 | 9.83463 | 100.0 | 364 | 21035 | 0.174 |
| 9.916 | 8.90757 | 8.7 | 32.3 | 1832 | 0.168 |
| 11.301 | 7.81865 | 27.8 | 104 | 5844 | 0.167 |
| 14.521 | 6.09321 | 20.2 | 75.5 | 4251 | 0.108 |
| 14.902 | 5.93864 | 27.8 | 102 | 5850 | 0.162 |
| 15.93 | 5.56039 | 1.9 | 7.2 | 394 | 0.148 |
| 16.947 | 5.23254 | 1.2 | 4.96 | 253 | 0.169 |
| 17.581 | 5.04332 | 6.8 | 24.8 | 1429 | 0.182 |
| 18.046 | 4.91261 | 48.7 | 184 | 10242 | 0.189 |
| 18.863 | 4.70152 | 2.6 | 9.41 | 545 | 0.159 |
| 19.418 | 4.5672 | 31.6 | 115 | 6637 | 0.166 |
| 19.943 | 4.44853 | 9.3 | 33.8 | 1966 | 0.181 |
| 21.31 | 4.1658 | 41.4 | 151 | 8714 | 0.192 |
| 22.797 | 3.89704 | 9.1 | 33.2 | 1921 | 0.201 |
| 23.587 | 3.76939 | 14.7 | 53.1 | 3082 | 0.171 |
| 24.345 | 3.65381 | 19.6 | 71.2 | 4116 | 0.208 |
| 25.169 | 3.53603 | 2.3 | 8.44 | 479 | 0.185 |
| 25.895 | 3.43955 | 5.4 | 19.7 | 1129 | 0.152 |
| 26.221 | 3.39654 | 5.0 | 15.6 | 1061 | 0.146 |
| 26.689 | 3.33736 | 11.1 | 40 | 2329 | 0.192 |
| 27.249 | 3.27088 | 5.0 | 19.1 | 1052 | 0.25 |
| 28.09 | 3.17445 | 5.6 | 20.2 | 1184 | 0.269 |
| 28.886 | 3.08881 | 3.2 | 11.4 | 666 | 0.219 |
| 30.129 | 2.96435 | 4.2 | 15.6 | 884 | 0.184 |
| 30.585 | 2.92187 | 1.8 | 6.17 | 369 | 0.313 |
| 31.413 | 2.84617 | 5.6 | 20.1 | 1174 | 0.212 |
| 32.162 | 2.78029 | 2.8 | 9.87 | 583 | 0.285 |
| 33.878 | 2.64293 | 1.1 | 2.36 | 236 | 0.109 |
| 34.419 | 2.60386 | 3.2 | 11.5 | 676 | 0.239 |
| 35.529 | 2.52408 | 6.0 | 21.9 | 1254 | 0.344 |
| 36.598 | 2.45267 | 3.0 | 11 | 621 | 0.269 |
| 37.924 | 2.37119 | 1.8 | 6.41 | 371 | 0.276 |
| 38.818 | 2.31643 | 1.4 | 2.74 | 295 | 0.172 |
| 39.398 | 2.28753 | 1.1 | 3.56 | 236 | 0.196 |

**Table 2: Beta-lactam compounds**

| No. | IUPAC Name | CAS No. |
|---|---|---|
| 1 | (2S,5R,6R)-6-[(R)-2-(4-ethyl-2,3-dioxo-1-piperazinecarboxamido)-2-phenylacetamido]-3,3-dimethyl-7-oxo-4-thia-1-azabicyclo[3.2.0]heptane-2-carboxylic acid | 61477-96-1 |
| 2 | (2S,5R,6R)-3,3-dimethyl-7-oxo-6-(2-phenylacetamido)-4-thia-1-zabicyclo[3.2.0]heptane-2-carboxylic acid | 61-33-6 |
| 3 | (5R,6S)-6-[(1R)-1-hydroxyethyl]-3-({2-[(iminomethyl)amino]ethyl}thio)-7-oxo-1-azabicyclo[3.2.0]hept-2-ene-2-carboxylic acid | 74431-23-5 |
| 4 | (5R,6S)-6-((R)-1-hydroxyethyl)-7-oxo-3-((R)-tetrahydrofuran-2-yl)-4-thia-1-azabicyclo[3.2.0]hept-2-ene-2-carboxylic acid | 106560-14-9 |
| 5 | (2S,5R,6R)-6-{[3-(2-chlorophenyl)-5-methyl-oxazole-4-carbonyl]amino}-3,3-dimethyl-7-oxo-4-thia-1-azabicyclo[3.2.0]heptane-2-carboxylic acid | 61-72-3 |
| 6 | (6R,7R,Z)-7-(2-(2-aminothiazol-4-yl)-2-(2-carboxypropan-2-yloxyimino)acetamido)-8-oxo-3-(pyridinium-1-ylmethyl)-5-thia-1-aza-bicyclo[4.2.0] oct-2-ene-2-carboxylate | 72558-82-8 |
| 7 | (6R,7R,Z)-3-(acetoxymethyl)-7-(2-(2-aminothiazol-4-yl)-2-(methoxyimino)acetamido)-8-oxo-5-thia-1-azabicyclo[4.2.0]oct-2-ene-2-carboxylic acid | 63527-52-6 |
| 8 | (6R,7R)-7-[(2Z)-2-ethoxyimino-2-[5-(phosphonoamino)-1,2,4-thiadiazol-3-yl]acetyl]amino]-3-[4-(1-methylpyridin-1-ium-4-yl)-1,3-thiazol-2-yl]sulfanyl]-8-oxo-5-thia-1-azabicyclo[4.2.0]oct-2-ene-2-carboxylate | 400827-46-5 |
| 9 | (6R,7R,Z)-7-(2-(2-aminothiazol-4-yl)-2-(methoxyimino)acetamido)-3-((1-methylpyrrolidinium-1-yl)methyl)-8-oxo-5-thia-1-aza-bicyclo[4.2.0]oct-2-ene-2-carboxylate | 88040-23-7 |
| 10 | (6R,7R)-3-{[(aminocarbonyl)oxy]methyl}-7-{[(2Z)-2-(2-furyl)-2-(methoxyimino)acetyl]amino}-8-oxo-5-thia-1-azabicyclo[4.2.0]oct-2-ene-2-carboxylic acid | 55268-75-2 |
| 11 | (6R,7R)-7-{[(2Z)-2-(2-amino-1,3-thiazol-4-yl)-2-(methoxyimino)acetyl]amino}-3-{[(2-methyl-5,6-dioxo-1,2,5,6-tetrahydro-1,2,4-triazin-3-yl)thio]methyl}-8-oxo-5-thia-1-azabicyclo[4.2.0]oct-2-ene-2-carboxylic acid | 73384-59-5 |
| 12 | (2S,5R,6R)-6-{[(2R)-2-amino-2-(4-hydroxyphenyl)-acetyl]amino}-3,3-dimethyl-7-oxo-4-thia-1-azabicyclo[3.2.0]heptane-2-carboxylic acid | 26787-78-0 |
| 13 | 3-[5-(dimethylcarbamoyl) pyrrolidin-2-yl] sulfanyl-6-(1-hydroxyethyl)-4-methyl-7-oxo-1-azabicyclo[3.2.0]hept-2-ene-2-carboxylic acid | 119478-56-7 |
| 14 | (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate | 689293-68-3 |
| 15 | 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate | 936111-69-2 |

**Table 3: Formulations of ceftolozane sulfate**

| **Component** | **Formulation A** | **Formulation B** | **Formulation C** | **Formulation D** |
|---|---|---|---|---|
| | grams (mmol) | grams (mmol) | grams (mmol) | grams (mmol) |
| ceftolozane sulfate | 1.00* (1.5) | 1.00* (1.5) | 1.00* (1.5) | 0.47 (0.70) |
| L-arginine | 0.62 (3.56) | 0.93 (5.34) | 0.62 (3.56) | 0.29 (1.67) |
| Citric acid | 0.022 | 0.022 | 0.022 | 0.01 |
| NaCl | 0.49 | 0.49 | 0.49 | 0.23 |
| Tazobactam acid | - | 0.50 (1.67) | - | - |
| Polymorph Ia | - | - | 0.79 (1.67) | - |
| Sodium tazobactam | - | - | - | 0.21* (0.65) |
| pH | 5.81 | 6.72 | 6.30 | 5.89 |

| | | | | |
|---|---|---|---|---|
| * active weight | | | | |

**Table 4: Stability data for formulations of Table 1 at 25 °C (60% RH), T1 (1 month), T2 (3 month)**

| HPLC Peaks | Formulation A | | | Formulation B | | | Formulation C | | | Formulation D | | |
|---|---|---|---|---|---|---|---|---|---|---|---|---|
| | T0 | T1 | T2 | T0 | T1 | T2 | T0 | T1 | T2 | T0 | T1 | T2 |
| Ceftolozane | 98.46% | 97.89% | 97.46% | 98.01% | 97.09% | 93.76% | 98.31% | 98.03% | 97.01% | 98.53% | 97.78% | 88.28% |
| Peak1 (RRT 0.150) | 0.29% | 0.42% | 0.80% | 0.39% | 0.85% | 2.57% | 0.30% | 0.46% | 0.83% | 0.21% | 0.45% | 6.08% |
| Peak3 (RRT 0.429) | 0.09% | 0.06% | 0.14% | 0.10% | 0.11% | 0.59% | 0.09% | 0.05% | 0.18% | 0.06% | 0.05% | 2.03% |
| Peak4 (RRT 0.612) | 0.05% | 0.08% | 0.09% | 0.06% | <0.03% | 0.13% | 0.06% | <0.03% | 0.08% | <0.03% | <0.03% | <0.03% |
| Peak5 (RRT 0.872) | 0.11% | 0.12% | 0.12% | 0.12% | 0.12% | <0.03% | 0.11% | 0.12% | 0.12% | 0.11% | 0.11% | <0.03% |
| Peak7 (RRT 1.262) | 0.89% | 0.90% | 0.96% | 0.88% | 0.95% | <0.03% | 0.89% | 0.85% | <0.03% | 0.88% | 0.92% | <0.03% |
| Peak8 (RRT 1.394) | <0.03% | <0.03% | <0.03% | 0.10% | <0.03% | <0.03% | 0.07% | <0.03% | <0.03% | <0.03% | <0.03% | <0.03% |
| Peak9 (RRT 1.684) | 0.04% | 0.04% | <0.03% | 0.04% | <0.03% | <0.03% | <0.03% | <0.03% | <0.03% | <0.03% | <0.03% | <0.03% |
| Others (RRT 0.120) | <0.03% | <0.03% | <0.03% | <0.03% | <0.03% | 0.15% | <0.03% | <0.03% | 0.04% | <0.03% | <0.03% | 0.42% |
| Others (RRT 0.653) | <0.03% | <0.03% | <0.03% | <0.03% | <0.03% | 0.10% | <0.03% | <0.03% | <0.03% | <0.03% | <0.03% | 0.28% |
| Others (RRT 0.904) | <0.03% | <0.03% | <0.03% | <0.03% | <0.03% | 0.12% | <0.03% | <0.03% | <0.03% | <0.03% | <0.03% | 0.68% |
| Others (RRT 1.22) | <0.03% | <0.03% | <0.03% | 0.05% | 0.38% | <0.03% | <0.03% | <0.03% | <0.03% | <0.03% | 0.17% | <0.03% |
| Others (RRT 1.255) | <0.03% | <0.03% | <0.03% | <0.03% | <0.03% | 1.59% | <0.03% | <0.03% | 1.18% | <0.03% | <0.03% | 1.82% |

## Claims

1. A pharmaceutical composition comprising crystalline tazobactam arginine and a beta-lactam compound which is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate or a pharmaceutically acceptable salt thereof.

2. A method of making a pharmaceutical composition comprising combining crystalline tazobactam arginine and a beta-lactam compound which is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate or a pharmaceutically acceptable salt thereof.

3. The method of claim 2, comprising the steps of:
(1) preparing a mixture comprising crystalline tazobactam arginine and the beta-lactam compound;
(2) preparing an aqueous solution from the mixture; and
(3) lyophilizing the solution to obtain said pharmaceutical composition.

4. The pharmaceutical composition or the method of any one of the preceding claims, wherein the crystalline tazobactam arginine is **characterized by** an X-ray powder diffraction pattern having one or more characteristic peaks expressed in degrees 2-Theta at angles selected from about 8.9° ± 0.3°, about 18.0° ± 0.3° and about 21.2° ± 0.3 or wherein the crystalline tazobactam arginine is **characterized by** an X-ray powder diffraction pattern having one or more characteristic peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°.

5. The pharmaceutical composition or the method of any one of the preceding claims, wherein the crystalline tazobactam arginine is **characterized by** a differential scanning calorimetry thermogram having a characteristic peak expressed in units of °C at a temperature in the range of about 209.2 to about 211.9, and/or wherein the crystalline tazobactam arginine is **characterized by** a thermogravimetry curve with an onset temperature of about 201.9 °C.

6. The pharmaceutical composition or the method of any one of the preceding claims, wherein the beta-lactam compound is 5-amino-4-{[(2-aminoethyl)carbamoyl]amino}-2-{[(6*R*,7*R*)-7-({(2*Z*)-2-(5-amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazolium monosulfate.

7. The method of any one of claims 2-3, wherein:
A) the molar ratio of crystalline tazobactam arginine to beta-lactam compound in the mixture is in the range of 1:2 to 2:1, such as about 0.9:1, or
B) the aqueous solution has a pH in the range of 5.5-6.5, such as about 6.3.

8. The method of any one of claims 2-3, wherein the mixture further comprises one or more additives selected from the list consisting of: L-arginine, citric acid, and sodium chloride, optionally wherein:
A) the molar ratio of L-arginine to beta-lactam compound in the mixture is in the range of 4:1 to 2:1, such as about 1.9:1, or
B) the concentration of the beta-lactam compound in the aqueous solution is in the range of 0.01M - 1M, such as about 0.05M.

9. A pharmaceutical composition prepared according to the method of any one of claims 2-3.

10. The pharmaceutical composition of any one of claims 1, 4-6 and 9 that is formulated for parenteral administration, optionally that is formulated for administration by intravenous injection or infusion.

11. A pharmaceutical composition of any one of claims 1, 4-6 and 9 for use in a method for the treatment of bacterial infections in a mammal, comprising administering to said mammal a therapeutically effective amount of a pharmaceutical composition of any one of claims 1, 4-6 and 9, optionally wherein the bacterial infection is caused by an extended-spectrum beta-lactamase-producing organism, or wherein the bacterial infection is caused by an antibiotic-resistant organism.

12. Crystalline tazobactam arginine and a beta-lactam compound which is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate or a pharmaceutically acceptable salt thereof for use in a method of treating a bacterial infection in a mammal.

13. Crystalline tazobactam arginine for use in a method of treating a bacterial infection in a mammal, comprising administration of crystalline tazobactam arginine in combination with a beta-lactam compound which is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate or a pharmaceutically acceptable salt thereof.

14. A beta-lactam compound for use in a method of treating a bacterial infection in a mammal, comprising administration of a beta-lactam compound which is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate or a pharmaceutically acceptable salt thereof in combination with crystalline tazobactam arginine.

15. Crystalline tazobactam arginine and a beta-lactam compound which is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate or a pharmaceutically acceptable salt thereof as a combined preparation for simultaneous, separate or sequential use in a method of treating a bacterial infection in a mammal.

16. The crystalline tazobactam arginine and beta-lactam compound, the crystalline tazobactam arginine, or the beta-lactam compound for use according to any one of claims 12-15 wherein the crystalline tazobactam arginine and/or beta-lactam compound is:
A) parenterally administered,
B) intravenously administered, or
C) administered as an infusion.

17. The crystalline tazobactam arginine and beta-lactam compound, the crystalline tazobactam arginine, or the beta-lactam compound for use according to any one of claims 12 to 16, wherein the bacterial infection is:
A) caused by an extended-spectrum beta-lactamase-producing organism,
B) caused by an antibiotic-resistant organism,
C) a complicated urinary tract infection,
D) a complicated intra-abdominal infection, or
E) nosocomial pneumonia.

18. The pharmaceutical composition, the crystalline tazobactam arginine, the beta-lactam compound, or the crystalline tazobactam arginine and beta-lactam compound for use according to any one of claims 11 to 17, wherein the beta-lactam compound is (6R,7R)-3-[(5-amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-ene-2-carboxylate, or a pharmaceutically acceptable salt, ester, hydrate, solvate, or combination thereof, optionally wherein the beta-lactam compound is 5-amino-4-{[(2-aminoethyl)carbamoyl]amino}-2-{[(6*R*,7*R*)-7-({(2*Z*)-2-(5-amino-1,2,4- thiadiazol-3-yl)-2-[(1 -carboxy-1 -methylethoxy)imino]acetyl} amino)-2-carboxy-8-oxo-5- thia-1-azabicyclo[4.2.0]oct-2-en-3-yl]methyl}-1-methyl-1*H*-pyrazolium monosulfate.

19. The pharmaceutical composition, the crystalline tazobactam arginine, the beta-lactam compound, or the crystalline tazobactam arginine and beta-lactam compound for use according to any one of claims 11 to 18, wherein the crystalline tazobactam arginine is **characterized by** an X-ray powder diffraction pattern having one or more characteristic peaks expressed in degrees 2-Theta at angles of about 8.9° ± 0.3°, about 18.0° ± 0.3° and about 21.2° ± 0.3°, or wherein the crystalline tazobactam arginine is **characterized by** an X-ray powder diffraction pattern having peaks expressed in degrees 2-Theta at angles of about 4.8° ± 0.3°, about 8.9° ± 0.3°, about 11.3° ± 0.3°, about 14.9° ± 0.3°, about 18.0° ± 0.3°, about 19.4° ± 0.3°, about 21.2° ± 0.3° about 22.8° ± 0.3° and about 24.3° ± 0.3°.

20. The pharmaceutical composition, the crystalline tazobactam arginine, the beta-lactam compound, or the crystalline tazobactam arginine and beta-lactam compound for use according to any one of claims 11 to 18, wherein the crystalline tazobactam arginine is **characterized by** a differential scanning calorimetry thermogram having a characteristic peak expressed in units of °C at a temperature in the range of about 209.2 to about 211.9, or wherein the crystalline tazobactam arginine is **characterized by** a thermogravimetry curve with an onset temperature of about 201.9 °C.

## Patentansprüche

1. Eine pharmazeutische Zusammensetzung, umfassend kristallines Tazobactam-Arginin und eine Beta-Lactam-Verbindung, die (6R,7R)-3-[(5-Amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-en-2-carboxylat oder ein pharmazeutisch annehmbares Salz davon ist.

2. Ein Verfahren zur Herstellung einer pharmazeutischen Zusammensetzung, umfassend das Kombinieren von kristallinem Tazobactam-Arginin und einer Beta-Lactam-Verbindung, die (6R,7R)-3-[(5-Amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-en-2-carboxylat oder ein pharmazeutisch annehmbares Salz davon ist.

3. Das Verfahren nach Anspruch 2, umfassend die Schritte:
(1) Herstellen einer Mischung, die kristallines Tazobactam-Arginin und die Beta-Lactam-Verbindung umfasst,
(2) Herstellen einer wässrigen Lösung aus der Mischung und
(3) Gefriertrocknen der Lösung, um die pharmazeutische Zusammensetzung zu erhalten.

4. Die pharmazeutische Zusammensetzung oder das Verfahren nach einem der vorhergehenden Ansprüche, wobei das kristalline Tazobactam-Arginin **gekennzeichnet ist durch** ein Röntgenpulverdiffraktogramm mit einem oder mehreren charakteristischen Peaks, angegeben in Grad 2-Theta, bei Winkeln ausgewählt aus etwa 8,9° ± 0,3°, etwa 18,0° ± 0,3° und etwa 21,2° ± 0,3°, oder wobei das kristalline Tazobactam-Arginin **gekennzeichnet ist durch** ein Röntgenpulverdiffraktogramm mit einem oder mehreren charakteristischen Peaks, angegeben in Grad 2-Theta, bei Winkeln von etwa 4,8° ± 0,3°, etwa 8,9° ± 0,3°, etwa 11,3° ± 0,3°, etwa 14,9° ± 0,3°, etwa 18,0° ± 0,3°, etwa 19,4° ± 0,3°, etwa 21,2° ± 0,3°, etwa 22,8° ± 0,3° und etwa 24.3° ± 0,3°.

5. Die pharmazeutische Zusammensetzung oder das Verfahren nach einem der vorhergehenden Ansprüche, wobei das kristalline Tazobactam-Arginin **gekennzeichnet ist durch** ein Dynamische-Differenzkalorimetrie-Thermogramm mit einem charakteristischen Peak, angegeben in °C-Einheiten, bei einer Temperatur im Bereich von etwa 209,2 bis etwa 211,9, und/oder wobei das kristalline Tazobactam-Arginin **gekennzeichnet ist durch** eine Thermogravimetriekurve mit einer Onset-Temperatur von etwa 201,9°C.

6. Die pharmazeutische Zusammensetzung oder das Verfahren nach einem der vorhergehenden Ansprüche, wobei die beta-Lactam-Verbindung 5-Amino-4-{[(2-aminoethyl)carbamoyl]amino}-2-{[(6*R*,7*R*)-7-({(2*Z*)-2-(5-Amino-1,2,4-thiadiazol-3-yl)-2-[(1-carboxy-1-methylethoxy)imino]acetyl}amino)-2-carboxy-8-oxo-5-thia-1-azabicyclo[4.2.0]oct-2-en-3-yl]methyl}-1-methyl-1*H*-pyrazoliummonosulfat ist.

7. Das Verfahren nach einem der Ansprüche 2 - 3, wobei:
A) das Molverhältnis von kristallinem Tazobactam-Arginin zur Beta-Lactam-Verbindung in der Mischung im Bereich von 1:2 bis 2:1 liegt, z.B. bei etwa 0,9:1, oder
B) die wässrige Lösung einen pH-Wert im Bereich von 5,5 - 6,5 besitzt, wie z.B. etwa 6,3.

8. Das Verfahren nach einem der Ansprüche 2 - 3, wobei die Mischung ferner ein oder mehrere Additive umfasst, ausgewählt aus der Liste bestehend aus: L-Arginin, Citronensäure und Natriumchlorid, gegebenenfalls wobei:
A) das Molverhältnis von L-Arginin zu Beta-Lactam-Verbindung in der Mischung im Bereich von 4:1 bis 2:1 liegt, z.B. bei etwa 1,9:1, oder
B) die Konzentration der Beta-Lactam-Verbindung in der wässrigen Lösung im Bereich von 0,01M - 1M liegt, z.B. bei etwa 0,05M.

9. Eine pharmazeutische Zusammensetzung, die gemäß dem Verfahren nach einem der Ansprüche 2-3 hergestellt ist.

10. Die pharmazeutische Zusammensetzung nach einem der Ansprüche 1, 4 - 6 und 9, die zur parenteralen Verabreichung formuliert ist, gegebenenfalls die zur Verabreichung durch intravenöse Injektion oder Infusion formuliert ist.

11. Eine pharmazeutische Zusammensetzung nach einem der Ansprüche 1, 4 - 6 und 9 zur Verwendung bei einem Verfahren zur Behandlung von Bakterieninfektionen bei einem Säuger, umfassend die Verabreichung einer therapeutisch wirksamen Menge einer pharmazeutischen Zusammensetzung nach einem der Ansprüche 1, 4 - 6 und 9 an den Säuger, wobei gegebenenfalls die Bakterieninfektion durch einen Beta-Lactamase mit erweitertem Spektrum erzeugenden Organismus hervorgerufen wird, oder wobei die Bakterieninfektion durch einen antibiotikaresistenten Organismus hervorgerufen wird.

12. Kristallines Tazobactam-Arginin und eine Beta-Lactam-Verbindung, die (6R,7R)-3-[(5-Amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-en-2-carboxylat oder ein pharmazeutisch annehmbares Salz davon ist, zur Verwendung bei einem Verfahren zur Behandlung einer Bakterieninfektion bei einem Säuger.

13. Kristallines Tazobactam-Arginin zur Verwendung bei einem Verfahren zur Behandlung einer Bakterieninfektion bei einem Säuger, umfassend die Verabreichung von kristallinem Tazobactam-Arginin in Kombination mit einer Beta-Lactam-Verbindung, die (6R,7R)-3-[(5-Amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-en-2-carboxylat oder ein pharmazeutisch annehmbares Salz davon ist.

14. Eine Beta-Lactam-Verbindung zur Verwendung bei einem Verfahren zur Behandlung einer Bakterieninfektion bei einem Säuger, umfassend die Verabreichung einer Beta-Lactam-Verbindung, die (6R,7R)-3-[(5-Amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-en-2-carboxylat oder ein pharmazeutisch annehmbares Salz davon ist, in Kombination mit kristallinem Tazobactam-Arginin.

15. Kristallines Tazobactam-Arginin und eine Beta-Lactam-Verbindung, die (6R,7R)-3-[(5-Amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1-carboxy-1-methylethoxy)imino]acetyl}amino)-8-oxo-5-thia-1-azabicyclo[4.2.0]oct-2-en-2-carboxylat oder ein pharmazeutisch annehmbares Salz davon ist, als ein Kombinationspräparat zur gleichzeitigen, separaten oder sequentiellen Verwendung bei einem Verfahren zur Behandlung einer Bakterieninfektion bei einem Säuger.

16. Das kristalline Tazobactam-Arginin und die Beta-Lactam-Verbindung, das kristalline Tazobactam-Arginin oder die Beta-Lactam-Verbindung zur Verwendung gemäß einem der Ansprüche 12 - 15, wobei das kristalline Tazobactam-Arginin und/oder die Beta-Lactam-Verbindung:
A) parenteral verabreicht wird/werden,
B) intravenös verabreicht wird/werden oder
C) als Infusion verabreicht wird/werden.

17. Das kristalline Tazobactam-Arginin und die Beta-Lactam-Verbindung, das kristalline Tazobactam-Arginin oder die Beta-Lactam-Verbindung zur Verwendung gemäß einem der Ansprüche 12 bis 16, wobei die Bakterieninfektion:
A) durch einen Beta-Lactamase mit erweitertem Spektrum erzeugenden Organismus hervorgerufen wird,
B) durch einen antibiotikaresistenten Organismus hervorgerufen wird,
C) eine komplizierte Harnwegsinfektion ist,
D) eine komplizierte intraabdominale Infektion ist oder
E) nosokomiale Pneumonie ist.

18. Die pharmazeutische Zusammensetzung, das kristalline Tazobactam-Arginin, die Beta-Lactam-Verbindung oder das kristalline Tazobactam-Arginin und die Beta-Lactam-Verbindung zur Verwendung gemäß einem der Ansprüche 11 bis 17, wobei die Beta-Lactam-Verbindung (6R,7R)-3-[(5-Amino-4-{[(2-aminoethyl)carbamoyl]amino}-1-methyl-1H-pyrazol-2-ium-2-yl)methyl]-7-({(2Z)-2-(5-amino-1,2,4-thiadiazol-3-yl)-2-[(1iazol-3-yl)-2-[(1-carboxy-1-methylethoxy)imino]acetyl}amino)-8-oxo-5-thia-1-azabicyclo[4.2.0]oct-2-en-2-carboxylat oder ein pharmazeutisch annehmbares/r Salz, Ester, Hydrat, Solvat oder eine Kombination davon ist, gegebenenfalls wobei die Beta-Lactam-Verbindung 5-Amino-4-{[(2-aminoethyl)carbamoyl]amino}-2-{[(6*R*,7*R*)-7-({(2*Z*)-2-(5-Amino-1,2,4-thiadiazol-3-yl)-2-[(1-carboxy-1-methyiethoxy)imino]acetyl}amino)-2-carboxy-8-oxo-5-thia-1-azabicyclo[4.2.0]oct-2-en-3-yl]methyl}-1-methyl-1*H*-pyrazoliummonosulfat ist.

19. Die pharmazeutische Zusammensetzung, das kristalline Tazobactam-Arginin, die Beta-Lactam-Verbindung oder das kristalline Tazobactam-Arginin und die Beta-Lactam-Verbindung zur Verwendung gemäß einem der Ansprüche 11 bis 18, wobei das kristalline Tazobactam-Arginin **gekennzeichnet ist durch** ein Röntgenpulverdiffraktogramm mit einem oder mehreren charakteristischen Peaks, angegeben in Grad 2-Theta, bei Winkeln von etwa 8,9° ± 0,3°, etwa 18,0° ± 0,3° und etwa 21,2° ± 0,3°, oder wobei das kristalline Tazobactam-Arginin **gekennzeichnet ist durch** ein Röntgenpulverdiffraktogramm mit Peaks, angegeben in Grad 2-Theta, bei Winkeln von etwa 4,8° ± 0,3°, etwa 8,9° ± 0,3°, etwa 11,3° ± 0,3°, etwa 14,9° ± 0,3°, etwa 18,0° ± 0,3°, etwa 19,4° ± 0,3°, etwa 21,2° ± 0,3°, etwa 22,8° ± 0,3° und etwa 24.3° ± 0,3°.

20. Die pharmazeutische Zusammensetzung, das kristalline Tazobactam-Arginin, die Beta-Lactam-Verbindung oder das kristalline Tazobactam-Arginin und die Beta-Lactam-Verbindung zur Verwendung gemäß einem der Ansprüche 11 bis 18, wobei das kristalline Tazobactam-Arginin **gekennzeichnet ist durch** ein Dynamische-Differenzkalorimetrie-Thermogramm mit einem charakteristischen Peak, angegeben in °C-Einheiten, bei einer Temperatur im Bereich von etwa 209,2 bis etwa 211,9, oder wobei das kristalline Tazobactam-Arginin **gekennzeichnet ist durch** eine Thermogravimetriekurve mit einer Onset-Temperatur von etwa 201,9°C.

## Revendications

1. Composition pharmaceutique comprenant une arginine-tazobactame cristalline et un composé de bêta-lactame qui est le (6R,7R)-3-[(5-amino-4-{[(2-aminoéthyl)carbamoyl]amino}-1-méthyl-1H-pyrazol-2-ium-2-yl)méthyl]-7-({(2Z)-2-(5-amino-1,2,4-thiadiazol-3-yl)-2-[(1-carboxy-1-méthyléthoxy)imino]acétyl}amino)-8-oxo-5-thia-1-azabicyclo[4.2.0]oct-2-ène-2-carboxylate ou un sel pharmaceutiquement acceptable de celui-ci.

2. Méthode pour la fabrication d'une composition pharmaceutique comprenant la combinaison d'une arginine-tazobactame cristalline et d'un composé de bêta-lactame qui est le (6R,7R)-3-[(5-amino-4-{[(2-aminoéthyl)carbamoyl]amino}-1-méthyl-1H-pyrazol-2-ium-2-yl)méthyl]-7-({(2Z)-2-(5-amino-1,2,4-thiadiazol-3-yl)-2-[(1-carboxy-1-méthyléthoxy)imino]acétyl}amino)-8-oxo-5-thia-1-azabicyclo[4.2.0]oct-2-ène-2-carboxylate ou un sel pharmaceutiquement acceptable de celui-ci.

3. Méthode selon la revendication 2, comprenant les étapes de:
(1) préparation d'un mélange comprenant une arginine-tazobactame cristalline et le composé de bêta-lactame;
(2) préparation d'une solution aqueuse à partir du mélange; et
(3) lyophilisation de la solution pour obtenir ladite composition pharmaceutique.

4. Composition pharmaceutique ou méthode selon l'une quelconque des revendications précédentes, dans laquelle l'arginine-tazobactame cristalline est **caractérisée par** un diagramme de diffraction des rayons X de poudre possédant un ou plusieurs pics caractéristiques exprimés en degrés 2-Thêta à des angles choisis parmi environ 8,9° ± 0,3°, environ 18,0° ± 0,3° et environ 21,2° ± 0,3° ou dans laquelle l'arginine-tazobactame cristalline est **caractérisée par** un diagramme de diffraction des rayons X de poudre possédant un ou plusieurs pics caractéristiques exprimés en degrés 2-Thêta à des angles d'environ 4,8° ± 0,3°, environ 8,9° ± 0,3°, environ 11,3° ± 0,3°, environ 14,9° ± 0,3°, environ 18,0° ± 0,3°, environ 19,4° ± 0,3°, environ 21,2° ± 0,3°, environ 22,8° ± 0,3° et environ 24,3° ± 0,3°.

5. Composition pharmaceutique ou méthode selon l'une quelconque des revendications précédentes, dans laquelle l'arginine-tazobactame cristalline est **caractérisée par** un thermogramme de calorimétrie différentielle à balayage possédant un pic caractéristique exprimé en unités de °C à une température dans l'intervalle d'environ 209,2 à environ 211,9 et/ou dans laquelle l'arginine-tazobactame cristalline est **caractérisée par** une courbe de thermogravimétrie avec une température de départ d'environ 201,9°C.

6. Composition pharmaceutique ou méthode selon l'une quelconque des revendications précédentes, dans laquelle le composé de bêta-lactame est le monosulfate de 5-amino-4-{[(2-aminoéthyl)carbamoyl]amino}-2-{[(6R,7R)-7-({(2Z)-2-(5-amino-1,2,4-thiadiazol-3-yl)-2-[(1-carboxy-1-méthyléthoxy)imino]acétyl}amino)-2-carboxy-8-oxo-5-thia-1-azabicyclo[4.2.0]oct-2-én-3-yl]méthyl}-1-méthyl-1H-pyrazolium.

7. Méthode selon l'une quelconque des revendications 2-3, dans laquelle:
A) le rapport molaire de l'arginine-tazobactame cristalline sur le composé de bêta-lactame dans le mélange est dans l'intervalle de 1:2 à 2:1, tel qu'environ 0,9:1, ou
B) la solution aqueuse a un pH dans l'intervalle de 5,5-6,5, tel qu'environ 6,3.

8. Méthode selon l'une quelconque des revendications 2-3, dans laquelle le mélange comprend en outre un ou plusieurs additifs choisis dans la liste constituée de: L-arginine, acide citrique et chlorure de sodium, optionnellement dans laquelle:
A) le rapport molaire de la L-arginine sur le composé de bêta-lactame dans le mélange est dans l'intervalle de 4:1 à 2:1, tel qu'environ 1,9:1, ou
B) la concentration du composé de bêta-lactame dans la solution aqueuse est dans l'intervalle de 0,01 M-1 M, tel qu'environ 0,05 M.

9. Composition pharmaceutique préparée selon la méthode de l'une quelconque des revendications 2-3.

10. Composition pharmaceutique selon l'une quelconque des revendications 1, 4-6 et 9 qui est formulée pour une administration parentérale, optionnellement qui est formulée pour une administration par une injection ou une perfusion intraveineuse.

11. Composition pharmaceutique selon l'une quelconque des revendications 1, 4-6 et 9 pour une utilisation dans une méthode pour le traitement d'infections bactériennes chez un mammifère, comprenant l'administration audit mammifère d'une quantité thérapeutiquement efficace d'une composition pharmaceutique selon l'une quelconque des revendications 1, 4-6 et 9, optionnellement dans laquelle l'infection bactérienne est entraînée par un organisme produisant une bêta-lactamase à spectre étendu ou dans laquelle l'infection bactérienne est entraînée par un organisme résistant aux antibiotiques.

12. Arginine-tazobactame cristalline et un composé de bêta-lactame qui est le (6R,7R)-3-[(5-amino-4-{[(2-aminoéthyl)carbamoyl]amino}-1-méthyl-1H-pyrazol-2-ium-2-yl)méthyl]-7-({(2Z)-2-(5-amino-1,2,4-thiadiazol-3-yl)-2-[(1-carboxy-1-méthyléthoxy)imino]acétyl}amino)-8-oxo-5-thia-1-azabicyclo[4.2.0]oct-2-ène-2-carboxylate ou un sel pharmaceutiquement acceptable de celui-ci pour une utilisation dans une méthode de traitement d'une infection bactérienne chez un mammifère.

13. Arginine-tazobactame cristalline pour une utilisation dans une méthode de traitement d'une infection bactérienne chez un mammifère, comprenant l'administration d'une arginine-tazobactame cristalline en combinaison avec un composé de bêta-lactame qui est le (6R,7R)-3-[(5-amino-4-{[(2-aminoéthyl)carbamoyl]amino}-1-méthyl-1H-pyrazol-2-ium-2-yl)méthyl]-7-({(2Z)-2-(5-amino-1,2,4-thiadiazol-3-yl)-2-[(1-carboxy-1-méthyléthoxy)imino]acétyl}amino)-8-oxo-5-thia-1-azabicyclo[4.2.0]oct-2-ène-2-carboxylate ou un sel pharmaceutiquement acceptable de celui-ci.

14. Composé de bêta-lactame pour une utilisation dans une méthode de traitement d'une infection bactérienne chez un mammifère, comprenant l'administration d'un composé de bêta-lactame qui est le (6R,7R)-3-[(5-amino-4-{[(2-aminoéthyl)carbamoyl]amino}-1-méthyl-1H-pyrazol-2-ium-2-yl)méthyl]-7-({(2Z)-2-(5-amino-1,2,4-thiadiazol-3-yl)-2-[(1-carboxy-1-méthyléthoxy)imino]acétyl}amino)-8-oxo-5-thia-1-azabicyclo[4.2.0]oct-2-ène-2-carboxylate ou un sel pharmaceutiquement acceptable de celui-ci en combinaison avec une arginine-tazobactame cristalline.

15. Arginine-tazobactame cristalline et un composé de bêta-lactame qui est le (6R,7R)-3-[(5-amino-4-{[(2-aminoéthyl)carbamoyl]amino}-1-méthyl-1H-pyrazol-2-ium-2-yl)méthyl]-7-({(2Z)-2-(5-amino-1,2,4-thiadiazol-3-yl)-2-[(1-carboxy-1-méthyléthoxy)imino]acétyl}amino)-8-oxo-5-thia-1-azabicyclo[4.2.0]oct-2-ène-2-carboxylate ou un sel pharmaceutiquement acceptable de celui-ci sous forme d'une préparation combinée pour une utilisation simultanée, séparée ou séquentielle dans une méthode de traitement d'une infection bactérienne chez un mammifère.

16. Arginine-tazobactame cristalline et un composé de bêta-lactame, arginine-tazobactame cristalline ou composé de bêta-lactame pour une utilisation selon l'une quelconque des revendications 12-15, où l'arginine-tazobactame cristalline et/ou le composé de bêta-lactame est:
A) administré par voie parentérale,
B) administré par voie intraveineuse, ou
C) administré comme une perfusion.

17. Arginine-tazobactame cristalline et un composé de bêta-lactame, arginine-tazobactame cristalline ou composé de bêta-lactame pour une utilisation selon l'une quelconque des revendications 12 à 16, où l'infection bactérienne est:
A) entraînée par un organisme produisant une bêta-lactamase à spectre étendu,
B) entraînée par un organisme résistant aux antibiotiques,
C) une infection du tractus urinaire compliquée,
D) une infection intra-abdominale compliquée, ou
E) une pneumonie nosocomiale.

18. Composition pharmaceutique, arginine-tazobactame cristalline, composé de bêta-lactame ou arginine-tazobactame cristalline et composé de bêta-lactame pour une utilisation selon l'une quelconque des revendications 11 à 17, dans laquelle le composé de bêta-lactame est le (6R,7R)-3-[(5-amino-4-{[(2-aminoéthyl)carbamoyl]amino}-1-méthyl-1H-pyrazol-2-ium-2-yl)méthyl]-7-({(2Z)-2-(5-amino-1,2,4-thiadiazol-3-yl)-2-[(1-carboxy-1-méthyléthoxy)imino]acétyl}amino)-8-oxo-5-thia-1-azabicyclo[4.2.0]oct-2-ène-2-carboxylate ou un sel pharmaceutiquement acceptable, un ester, un hydrate, un solvate ou une combinaison de ceux-ci, optionnellement dans laquelle le composé de bêta-lactame est le monosulfate de 5-amino-4-{[(2-aminoéthyl)carbamoyl]amino}-2-{[(6R,7R)-7-({(2Z)-2-(5-amino-1,2,4-thiadiazol-3-yl)-2-[(1-carboxy-1-méthyléthoxy)imino]acétyl}amino)-2-carboxy-8-oxo-5-thia-1-azabicyclo[4.2.0]oct-2-én-3-yl]méthyl}-1-méthyl-1H-pyrazolium.

19. Composition pharmaceutique, arginine-tazobactame cristalline, composé de bêta-lactame ou arginine-tazobactame cristalline et composé de bêta-lactame pour une utilisation selon l'une quelconque des revendications 11 à 18, dans laquelle l'arginine-tazobactame cristalline est **caractérisée par** un diagramme de diffraction des rayons X de poudre possédant un ou plusieurs pics caractéristiques exprimés en degrés 2-Thêta à des angles d'environ 8,9° ± 0,3°, environ 18,0° ± 0,3° et environ 21,2° ± 0,3° ou dans laquelle l'arginine-tazobactame cristalline est **caractérisée par** un diagramme de diffraction des rayons X de poudre possédant des pics exprimés en degrés 2-Thêta à des angles d'environ 4,8° ± 0,3°, environ 8,9° ± 0,3°, environ 11,3° ± 0,3°, environ 14,9° ± 0,3°, environ 18,0° ± 0,3°, environ 19,4° ± 0,3°, environ 21,2° ± 0,3°, environ 22,8° ± 0,3° et environ 24,3° ± 0,3°.

20. Composition pharmaceutique, arginine-tazobactame cristalline, composé de bêta-lactame ou arginine-tazobactame cristalline et composé de bêta-lactame pour une utilisation selon l'une quelconque des revendications 11 à 18, dans laquelle l'arginine-tazobactame cristalline est **caractérisée par** un thermogramme de calorimétrie différentielle à balayage possédant un pic caractéristique exprimé en unités de °C à une température dans l'intervalle d'environ 209,2 à environ 211,9 ou dans laquelle l'arginine-tazobactame cristalline est **caractérisée par** une courbe de thermogravimétrie avec une température de départ d'environ 201,9°C.
